# EUROPÄISCHE PATENTSCHRIFT

(11) **EP 1 904 470 B1**
(45) Veröffentlichungstag und Bekanntmachung des Hinweises auf die Patenterteilung: **26.08.2009**
(21) Anmeldenummer: 06762522.8
(22) Anmeldetag: 11.07.2006
(51) Int. Cl.: C07D 317/70, C07C 22/00, C09K 19/32, C09K 19/34

(54) **S-INDACEN- UND INDENO[5,6-D][1,3]DIOXOL-DERIVATE UND DEREN VERWENDUNG ALS KOMPONENTE FLÜSSIGKRISTALLINER MISCHUNGEN**
S-INDACENE- AND INDENO[5,6-D][1,3]DIOXOL-DERIVATIVES AND USE THEREOF AS COMPONENTS OF LIQUID-CRYSTAL MIXTURES
DERIVES DE S-INDACENE- ET D'INDENO[5,6-D][1,3]DIOXOL ET LEUR UTILISATION COMME COMPOSANTS DE MELANGES A CRISTAUX LIQUIDES

(30) Priorität: 20.07.2005 DE 102005033803
(43) Veröffentlichungstag der Anmeldung: 02.04.2008
(73) Patentinhaber: Merck Patent GmbH, 64293 Darmstadt (DE)
(72) Erfinder: CZANTA, Markus, 64289 Darmstadt (DE); PAULUTH, Detlef, 64372 Ober-Ramstadt (DE); LIETZAU, Lars, 64295 Darmstadt (DE)
(86) Internationale Anmeldenummer: PCT/EP2006/006771
(87) Internationale Veröffentlichungsnummer: WO 2007/009636

(56) Entgegenhaltungen:
- WO-A-03/010120
- WO-A-20/04020375
- DE-A1- 3 908 269

## Beschreibung

Die Erfindung betrifft 1,2,3,6,7,8-Hexahydro-s-indacen-Derivate und 6,7-Dihydro-5H-indeno[5,6-d][1,3]dioxol-Derivate sowie ihre Verwendung als Komponente(n) in flüssigkristallinen Medien. Darüber hinaus betrifft die vorliegende Erfindung Flüssigkristall- und elektrooptische Anzeigeelemente, welche die erfindungsgemäßen, flüssigkristallinen Medien enthalten.

Die erfindungsgemäßen Verbindungen können als Komponente(n) flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (electrically controlled birefringence), dem IPS-Effekt (in-plane switching) oder dem Effekt der dynamischen Streuung beruhen.

In der Schrift DE 10135499 A1 ist ein 1,2,3,6,7,8-Hexahydro-s-indacengerüst mit Substituenten in Position 1,7 und 8 offenbart. Die Substanzen besitzen ausnahmslos negative Werte der dielektrischen Anisotropie Δε.
Die Schrift DE 3908269 A1 offenbart 1,2,3,6,7,8-Hexahydro-s-indacene, die in Position 2 und 6 jeweils genau einen Substituenten tragen.
Daneben offenbart die Patentanmeldung EP 1350780 A1 1,7-Dihydro-s-indacene mit mehreren optionalen Substituenten, die so platziert sind, dass ebenfalls nur Verbindungen mit negativen Werten der dielektrischen Anisotropie Δε betroffen sind.

Der vorliegenden Erfindung lag die Aufgabe zugrunde, neue und stabile Verbindungen aufzufinden, die als Komponenten flüssigkristalliner Medien geeignet sind. Insbesondere sollen die Verbindungen gleichzeitig eine vergleichsweise geringe Viskosität besitzen sowie eine dielektrische Anisotropie im positiven Bereich besitzen. Für viele aktuelle Mischungskonzepte im Bereich der Flüssigkristalle ist es vorteilhaft, Verbindungen mit einer besonders hohen dielektrischen Anisotropie Δε zu verwenden.

Im Hinblick auf die verschiedensten Einsatzbereiche derartiger Verbindungen mit hohem Δε war es wünschenswert, weitere Verbindungen, vorzugsweise mit hoher Nematogenität zur Verfügung zu haben, die auf die jeweiligen Anwendungen genau maßgeschneiderte Eigenschaften aufweisen.

Der Erfindung lag somit als eine Aufgabe zugrunde, neue stabile, Verbindungen aufzufinden, die als Komponente(n) flüssigkristalliner Medien, insbesondere für TN-, STN-, IPS- und weitere TFT-Displays, geeignet sind.

Eine weitere Aufgabe der vorliegenden Erfindung war es, Verbindungen bereitzustellen, die eine hohe dielektrische Anisotropie Δε, einen hohen Klärpunkt sowie eine niedrige Rotationsviskosität γ₁ aufweisen. Darüber hinaus sollten die erfindungsgemäßen Verbindungen thermisch und photochemisch stabil sein. Ferner sollten die erfindungsgemäßen Verbindungen in flüssigkristallinen Mischungen einsetzbar sein, indem sie sich in gebräuchlichen Mischungen lösen lassen und deren flüssigkristalline Phasenbereiche nicht beeinträchtigen oder gar verbessern.

Überraschenderweise wurde gefunden, dass die erfindungsgemäßen 1,2,3,6,7,8-Hexahydro-s-indacen- und 6,7-Dihydro-5H-indeno[5,6-d][1,3]dioxol-Derivate vorzüglich als Komponenten flüssigkristalliner Medien geeignet sind. Mit ihrer Hilfe lassen sich stabile, flüssigkristalline Medien, insbesondere geeignet für TN-TFT-, STN und IPS-Displays, erhalten. Die erfindungsgemäßen Verbindungen sind chemisch, thermisch und gegen (UV-)Licht stabil. Sie sind in reinem Zustand farblos. Auch zeichnen sie sich durch stark positive dielektrische Anisotropien Δε aus, aufgrund derer in der Anwendung in optischen Schaltelementen niedrigere Schwellenspannungen erforderlich sind. Darüber hinaus weisen die Verbindungen günstige, d. h. niedrige Werte für die Rotationsviskosität auf.

Flüssigkristalline Medien mit sehr kleinen Werten der optischen Anisotropie sind insbesondere für reflektive und transflektive Anwendungen von Bedeutung, d.h. solche Anwendungen, bei denen das jeweilige LCD keine oder nur unterstützende Hintergrundbeleuchtung erfährt. Danach ist es auch möglich, erfindungsgemäße Flüssigkristalle und Mischungen mit den erfindungsgemäßen Derivaten mit sehr kleinen Werten der optischen Anisotropie oder mit gering positiven bis stark positiven Werten der dielektrischen Anisotropie zu erhalten.

Mit der Bereitstellung der erfindungsgemäßen 1,2,3,6,7,8-Hexahydro-s-indacen- und 6,7-Dihydro-5H-indeno[5,6-d][1,3]dioxol-Derivate wird ganz allgemein die Palette der flüssigkristallinen Verbindungen, die sich unter verschiedenen, anwendungstechnischen Gesichtspunkten zur Herstellung flüssigkristalliner Mischungen eignen, erheblich verbreitert.

Die erfindungsgemäßen Verbindungen bilden in der Mischung mit geeigneten Cokomponenten flüssigkristalline Mesophasen in einem für die elektrooptische Anwendung günstig gelegenen Temperaturbereich. Flüssigkristalline Medien mit breiten nematischen Phasenbereichen lassen sich aus den erfindungsgemäßen Verbindungen und weiteren Substanzen herstellen.

Die 1,2,3,6,7,8-Hexahydro-s-indacen- und 6,7-Dihydro-5H-indeno[5,6-d][1,3]dioxol-Derivate besitzen einen breiten Anwendungsbereich. In Abhängigkeit von der Auswahl der Substituenten können diese Verbindungen als Basismaterialien dienen, aus denen flüssigkristalline Medien zum großen Teil zusammengesetzt sind. Es können aber auch den erfindungsgemäßen Verbindungen flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Gegenstand der vorliegenden Erfindung sind somit 1,2,3,6,7,8-Hexahydro-s-indacen- und 6,7-Dihydro-5H-indeno[5,6-d][1,3]dioxol-Derivate der allgemeinen Formel I 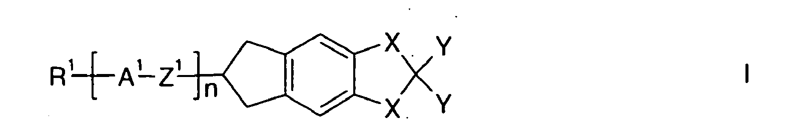 worin
- R¹: H, Halogen, einen linearen oder verzweigten, gegebenenfalls chiralen, unsubstituierten, einen einfach durch CN oder CF₃ substituierten oder einen mindestens einfach durch Halogen substituierten Alkylrest mit 1 bis 15 C-Atomen bedeutet, worin auch eine oder mehrere CH₂-Gruppen jeweils unabhängig voneinander durch -O-, -S-, -CO-, -(CO)O-, -CH=CH-, -CH=CF-, -CF=CF-, -C≡C-, -CH₂O- oder -CF₂O so ersetzt sein können, dass Heteroatome nicht direkt miteinander verknüpft sind und unsymmetrische Gruppen in beiden Orientierungen vorliegen können,
- A¹: jeweils unabhängig voneinander, gleich oder verschieden
a) trans-1,4-Cyclohexylen, worin auch eine oder mehrere nicht benachbarte CH₂-Gruppen durch -S- ersetzt sein können, Tetrahydropyran-2,5-diyl oder 1,3-Dioxan-2,5-diyl,
b) 1,4-Phenylen, worin eine oder zwei CH-Gruppen durch N ersetzt sein können und worin auch ein oder mehrere H-Atome gegen Halogen, bevorzugt durch F, ersetzt sein können,
c) einen Rest aus der Gruppe 1,4-Bicyclo(2,2,2)-octylen, spiro[3.3]heptan-2,6-diyl, Cyclobutan-1,3-diyl, Piperidin-1,4-diyl, Naphthalin-2,6-diyl, Decahydronaphthalin-2,6-diyl oder 1,2,3,4-Tetrahydronaphthalin-2,6-diyl, worin auch CH durch N oder auch ein oder mehrere H-Atome gegen Halogen, bevorzugt durch F, ersetzt sein können, oder
d) 1,4-Cyclohexenylen, wahlweise durch ein F substituiert,
- X: -CH₂-, -CF₂- oder -O-,
- Y: F, Cl, CF₃, CN, NCS, SCN, SF₅ oder 2- bis 6-C Perfluoralkyl,
- Z¹: jeweils unabhängig voneinander, bei unsymmetrischen Brückengliedern Z¹ wahlweise in beiden Orientierungen vorliegend,
eine Einfachbindung, -CH₂O-, -(CO)O-, -CF₂O-, -CF=CF--CH₂CH₂CF₂O-, -CF₂CF₂-, -CH₂CF₂-, -CH₂CH₂-, -CH=CH-, -CH=CF- oder -C≡C-, und
- n: 0, 1, 2 oder 3
bedeuten.

Ein weiterer Gegenstand der vorliegenden Erfindung ist die Verwendung von Verbindungen der Formel I als Komponente(n) in flüssigkristallinen Medien.

Ebenfalls Gegenstand der vorliegenden Erfindung sind flüssigkristalline Medien mit mindestens zwei flüssigkristallinen Komponenten, welche mindestens ein 1,2,3,6,7,8-Hexahydro-s-indacen- oder 6,7-Dihydro-5H-indeno[5,6-d][1,3]dioxol-Derivat der Formel I enthalten.

Gegenstand der vorliegenden Erfindung sind auch Flüssigkristall-Anzeigeelemente, insbesondere elektrooptische Anzeigeelemente, welche als Dielektrikum ein erfindungsgemäßes, flüssigkristallines Medium enthalten.

Die Bedeutung der Formel I schließt alle Isotope der in den Verbindungen der Formel I gebundenen chemischen Elemente ein. In enantiomerenreiner oder -angereicherter Form eignen sich die Verbindungen der Formel I prinzipiell auch als chirale Dotierstoffe und generell zur Erzielung chiraler Mesophasen.

Vor- und nachstehend haben R¹, A¹, Z¹, X, Y und n die angegebenen Bedeutungen, sofern nicht ausdrücklich etwas anderes vermerkt ist. Kommen die Reste A¹ und Z¹ mehrfach vor, so können sie, unabhängig voneinander, gleiche oder verschiedene Bedeutungen annehmen.

Der Einfachheit halber bedeutet im folgenden Cyc einen 1,4-Cyclohexyl-1,4-diylrest, Che einen (optional einfach fluorierten) Cyclohexen-1,4-diylrest, Dio einen 1,3-Dioxan-2,5-diylrest, Thp einen Tetrahydropyran-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest, Bco einen Bicyclo-(2,2,2)-octylenrest, Cbl ein 1,3-Cyclobutylen, Spi einen Spiro[3.3]heptan-2,6-diylrest und Dec einen Decahydronaphthalinrest, wobei Cyc und/oder Phe unsubstituiert bzw. ein- oder mehrfach durch F, Cl, CF₃, OCF₃ und/oder CN substituiert sein können.

Bevorzugt sind Verbindungen der Formel I, worin R¹ H, einen linearen Alkyl- bzw. Alkoxyrest mit 1 bis 12 C-Atomen oder einen linearen Alkenyl- bzw. Alkenyloxyrest mit 2 bis 12 C-Atomen bedeutet.

Ist R¹ Halogen, so bedeutet es vorzugsweise F oder Cl, besonders bevorzugt F.

A¹ bedeutet vorzugsweise Phe, Cyc, Che, Pyd, Pyr, Dio oder Thp und besonders bevorzugt Phe oder Cyc. Des weiteren bevorzugt sind Verbindungen der Formel I, die nicht mehr als einen der Reste Dio, Thp, Dit, Pyd, Pyr, Cbl, Spi oder Bco enthalten.

Ist der Ring A¹ zweimal vorhanden, so können die beiden Ringe gleiche oder verschiedene Bedeutungen haben. Dasselbe gilt auch für die Brücke Z¹.

Phe ist vorzugsweise

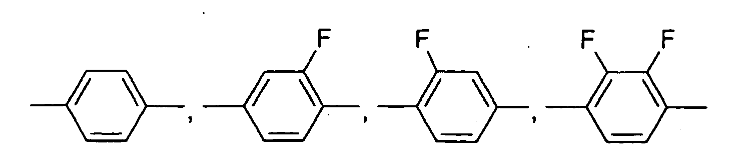

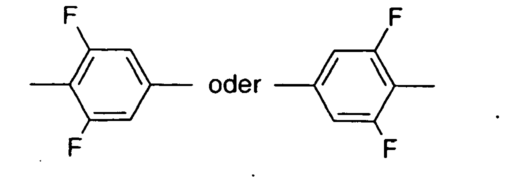

Phe ist besonders bevorzugt

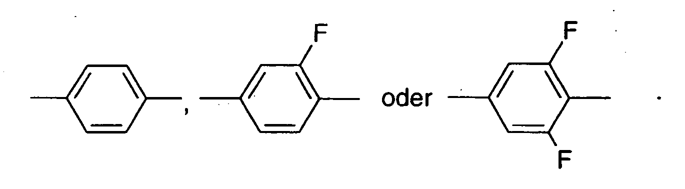

Die Begriffe 1,3-Dioxan-2,5-diyl und Dio umfassen jeweils die beiden Stellungsisomeren

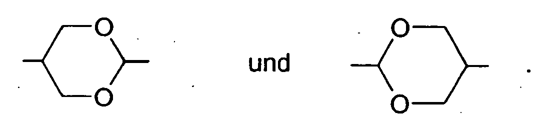

Die Begriffe Tetrahydropyran-2,5-diyl und Thp umfassen jeweils die beiden Stellungsisomeren

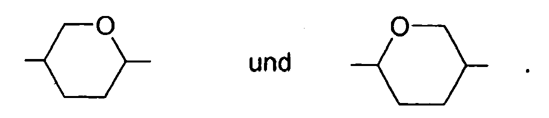

Die Cyclohexen-1,4-diyl-Gruppe (Che) hat vorzugsweise folgende Strukturen:

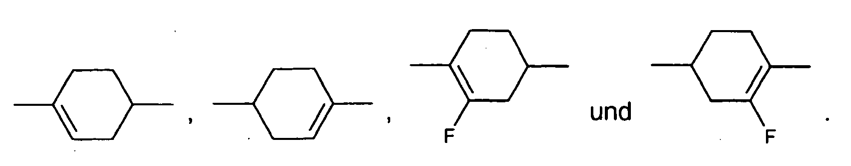

Y ist vorzugsweise F, CN, CF₃ oder OCF₃, besonders bevorzugt F oder CF₃.

Z¹ bedeutet vorzugsweise -CH₂CH₂-, -CH=CH-, -C≡C-, -CF₂CF₂-, -CF=CF-, -(CO)O-, -O(CO)-, -CF₂O- oder eine Einfachbindung, besonders bevorzugt -CF₂O-, -CH₂CH₂- oder eine Einfachbindung. Für den Fall, dass n > 1 ist, bedeutet vorzugsweise mindestens eines von Z¹ eine Einfachbindung.

n ist vorzugsweise 0, 1 oder 2, besonders bevorzugt 0 und 1. Wenn eines von Z¹ gleich der Gruppe -CF₂O- ist, dann ist n besonders bevorzugt 2 und der Molekülteil -[A¹-Z¹]ₙ- steht bevorzugt für -A¹-CF₂-O-Phe-, wobei Phe wie oben definiert ist.

Die erfindungsgemäßen Verbindungen der Formel I und ihrer untergeordneten Formeln lassen sich auch in der Form R¹-[A¹-Z¹]ₙ-W schreiben, wobei W für das kondensierte Ringsystem bestehend aus dem Molekülteil der Formel 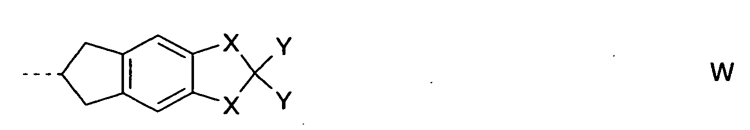 steht.

Bevorzugte Bedeutungen des Strukturelements W ergeben sich aus den bevorzugten Werten für X und Z. Besonders bevorzugte Kombinationen aus X und Z führen insbesondere zu den besonders bevorzugten Ausführungen des Molekülteils W der Teilformeln (1) bis (5):

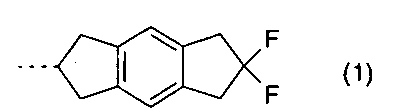

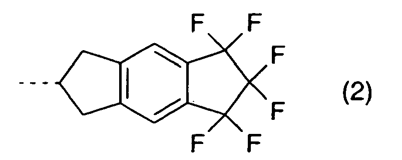

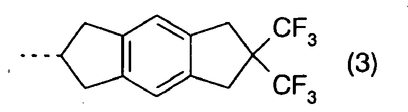

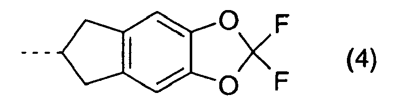

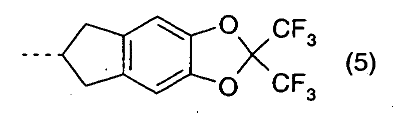

Die Verbindungen der Formel I umfassen neben dem Fall, dass n gleich 0 ist auch Verbindungen mit einem oder mehreren Ringen in der mesogenen Gruppe R¹-[A¹-Z¹]ₙ-, wie im Folgenden angeführt:
Verbindungen mit genau einem Ring in der mesogenen Gruppe R¹-[A¹-Z¹]ₙ- der Teilformeln Ia und Ib:

   R¹-A¹-W Ia

   R¹-A¹-Z¹-W Ib
Verbindungen mit zwei Ringen in der mesogenen Gruppe R¹-[A¹-Z¹]ₙ- der Teilformeln Ic bis If:

   R¹-A¹-A¹-W Ic

   R¹-A¹-A¹-Z¹-W Id

   R¹-A¹-Z¹-A¹-W Ie

   R¹-A¹-Z¹-A¹-Z¹-W If
Sowie Verbindungen mit drei Ringen in der mesogenen Gruppe R¹-[A¹-Z¹]ₙ- der Teilformeln Ig bis Io:

   R¹-A¹-A¹-A¹-W Ig

   R¹-A¹-Z¹-A¹-A¹-W Ih

   R¹-A¹-A¹-Z¹-A¹-W Ii

   R¹-A¹-A¹-A¹-Z¹-W Ij

   R¹-A¹-Z¹-A¹-Z¹-A¹-W Ik

   R¹-A¹-Z¹-A¹-A¹-Z¹-W Im

   R¹-A¹-A¹-Z¹-A¹-Z¹-W In

   R¹-A¹-Z¹-A¹-Z¹-A¹-Z¹-W Io

Darunter sind diejenigen der Teilformeln Ia, Ib, Ic, Id, le, Ig, Ih, li und Ij besonders bevorzugt, ganz besonders die der Formeln Ia, Ib, Ic, Id und Ii. Bevorzugt sind also die Formeln, in denen Z¹ immer eine Einfachbindung oder nur einmal keine Einfachbindung ist. Besonders bevorzugt ist gleichzeitig der Fall, dass n gleich 0 ist, also eine Verbindung der Formel R¹-W.

Die bevorzugten Verbindungen der Teilformel Ia umfassen diejenigen der Teilformeln laa bis laf:

R¹-Phe-W laa

R¹-Cyc-W lab

R¹-Thp-W lac

R¹-Dio-W Iad

R¹-Cbl-W lae

R¹-Spi-W laf

Darunter sind diejenigen der folgenden Teilformeln besonders bevorzugt:

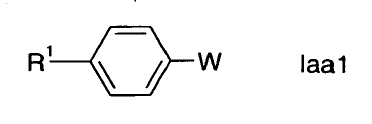

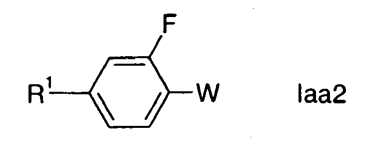

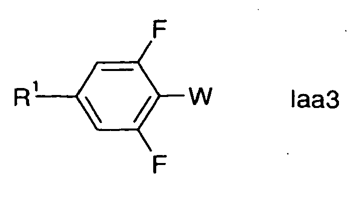

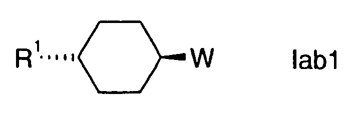

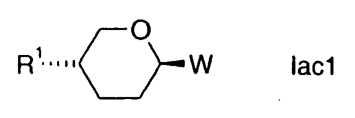

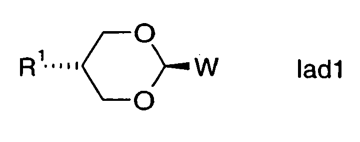

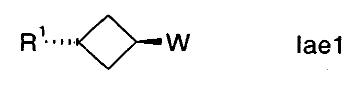

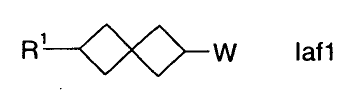

Die bevorzugten Verbindungen der Teilformel Ib umfassen diejenigen der Teilformeln Iba und Ibb:

R¹-Phe-Z¹-W Iba

R¹-Cyc-Z¹-W Ibb

Für die Verbindungen der Formeln Iba und Ibb ist Z¹ bevorzugt -CH₂CH₂-und Phe steht bevorzugt für ein 1,4-Phenylen.

Die bevorzugten Verbindungen der Teilformel Ic umfassen diejenigen der Teilformeln Ica bis Ico:

R¹-Cyc-Cyc-W Ica

R¹-Cyc-Thp-W Icb

R¹-Cyc-Dio-W Icc

R¹-Cyc-Phe-W Icd

R¹-Thp-Cyc-W Ice

R¹-Dio-Cyc-W Icf

R¹-Phe-Cyc-W Icg

R¹-Thp-Phe-W Ich

R¹-Dio-Phe-W Ici

R¹-Phe-Phe-W Icj

R¹-Pyr-Phe-W Ick

R¹-Thp-Dio-W Icm

R¹-Cbl-Cyc-W Icn

R¹-Spi-Cyc-W Ico

Darunter sind diejenigen der folgenden Teilformeln besonders bevorzugt:

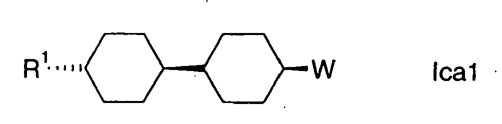

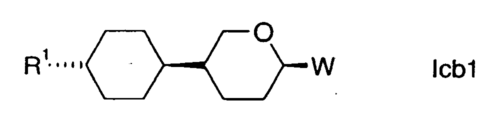

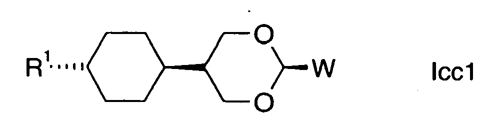

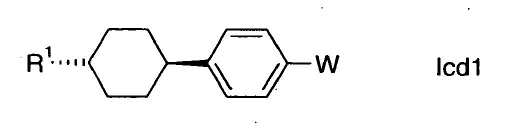

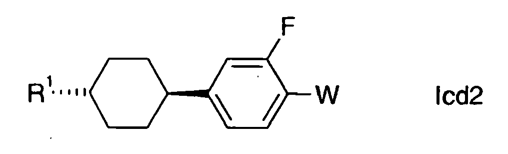

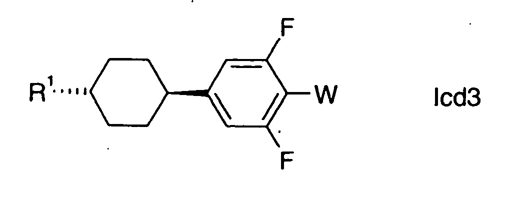

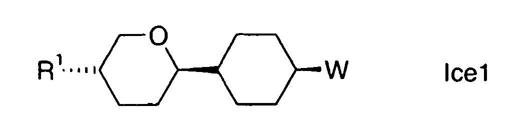

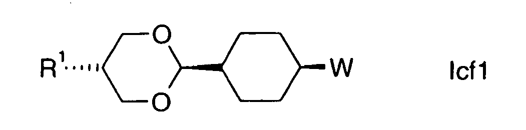

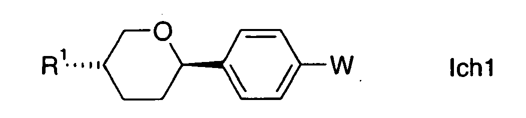

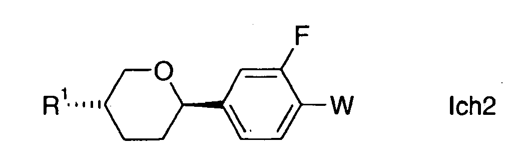

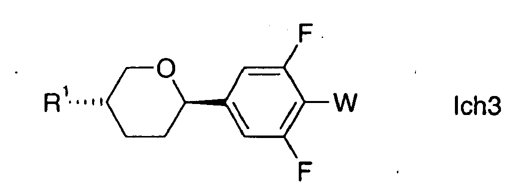

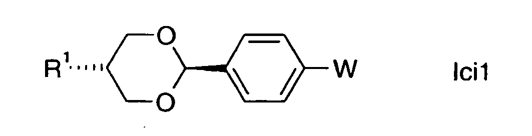

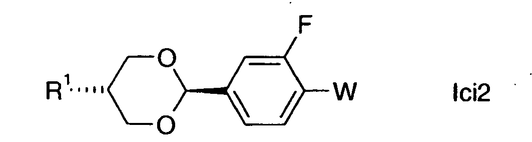

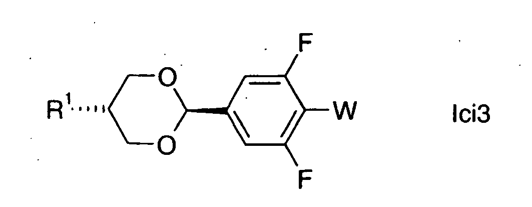

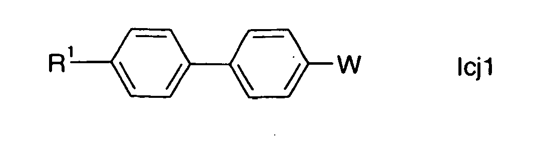

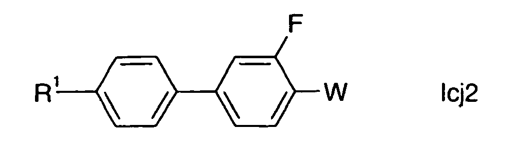

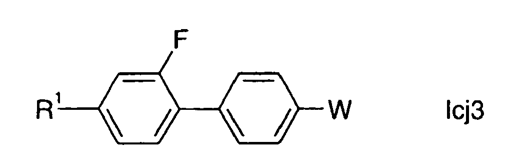

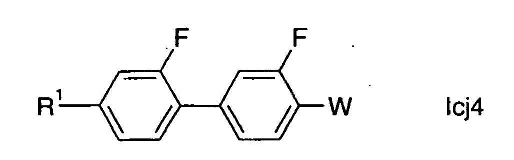

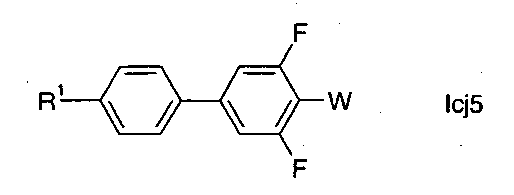

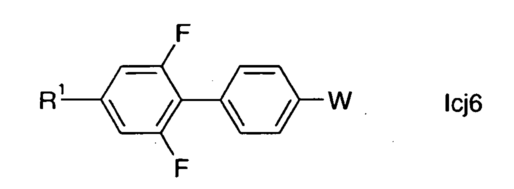

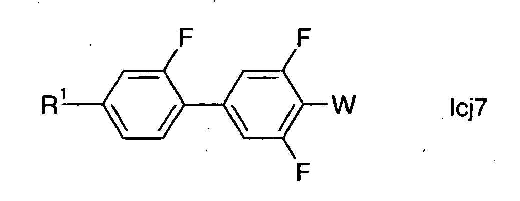

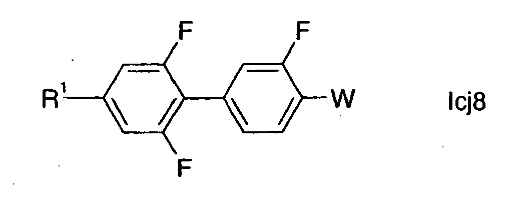

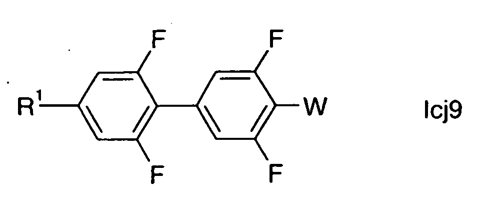

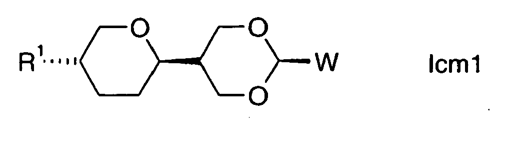

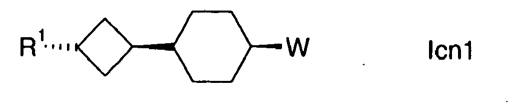

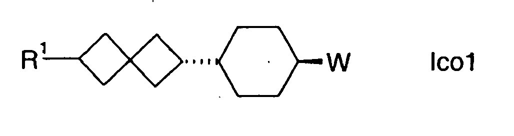

Die bevorzugten Verbindungen der Teilformel Id umfassen diejenigen der Teilformeln Ida bis Idp:

R¹-Cyc-Cyc-Z¹-W Ida

R¹-Cyc-Thp-Z¹-W Idb

R¹-Cyc-Dio-Z¹-W Idc

R¹-Cyc-Phe-Z¹-W Idd

R¹-Thp-Cyc-Z¹-W Ide

R¹-Dio-Cyc-Z¹-W Idf

R¹-Thp-Phe-Z¹-W Idg

R¹-Dio-Phe-Z¹-W Idh

R¹-Phe-Phe-Z¹-W Idi

R¹-Pyr-Phe-Z¹-W Idj

R¹-Pyd-Phe-Z¹-W Idk

R¹-Cyc-Phe-CH₂CH₂-W Idm

R¹-A¹-Phe-CH₂CH₂-W Idn

R¹-Cbl-Cyc-Z¹-W Ido

R¹-Spi-Cyc-Z¹-W Idp

Die bevorzugten Verbindungen der Teilformel le umfassen diejenigen der Teilformeln lea bis lem:

R¹-Cyc-Z¹-Cyc-W Iea

R¹-Thp-Z¹-Cyc-W leb

R¹-A¹-CH₂CH₂-A¹-W Iec

R¹-Cyc-Z¹-Phe-W Ied

R¹-Thp-Z¹-Phe-W lee

R¹-A¹-OCO-Phe-W lef

R¹-Phe-Z¹-Phe-W leg

R¹-Pyr-Z¹-A¹-W Ieh

R¹-Pyd-Z¹-A¹-W lei

R¹-Dio-Z¹-A¹-W lej

R¹-Cbl-Z¹-A¹-W lek

R¹-Spi-Z¹-A¹-W lem

Darunter sind diejenigen der folgenden Teilformeln besonders bevorzugt:

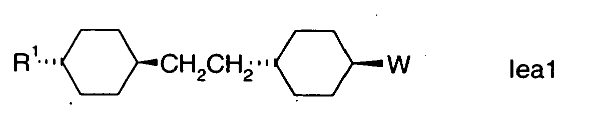

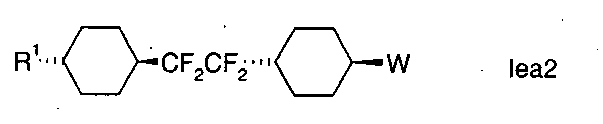

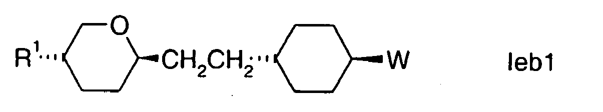

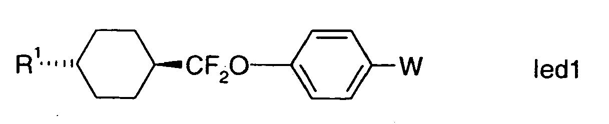

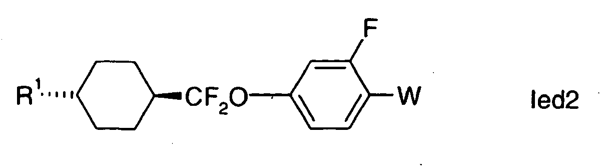

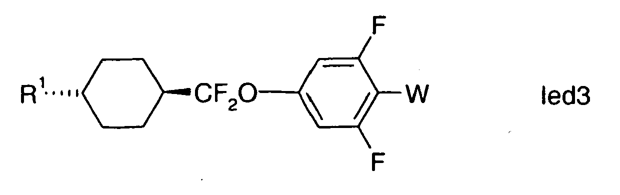

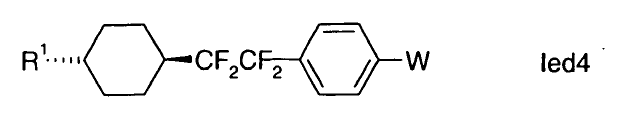

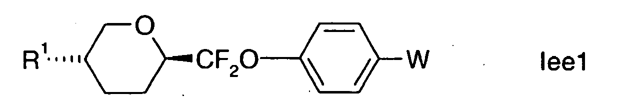

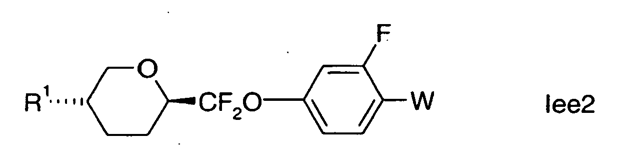

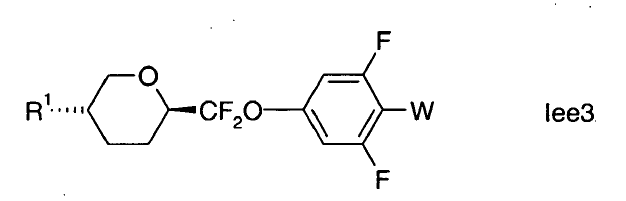

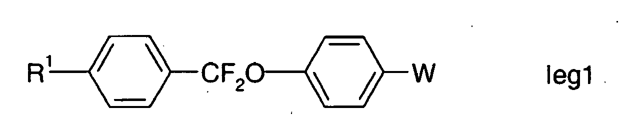

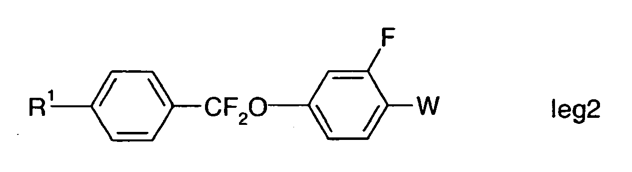

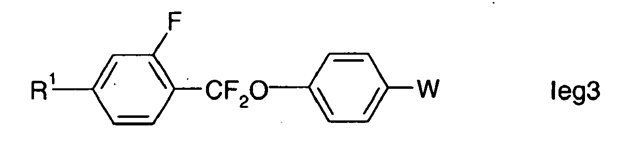

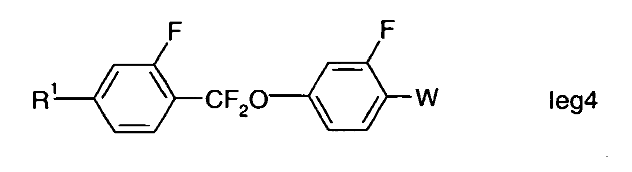

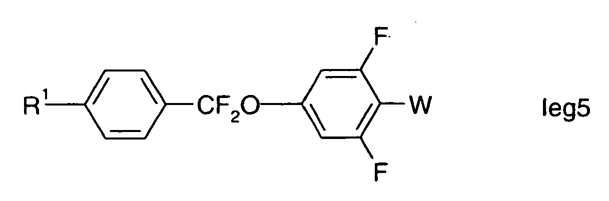

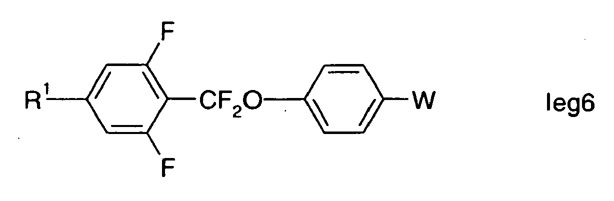

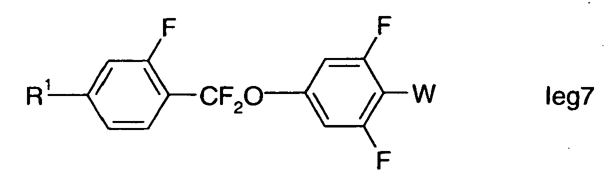

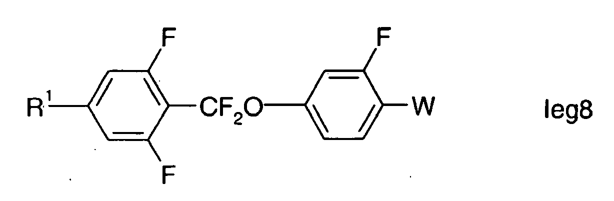

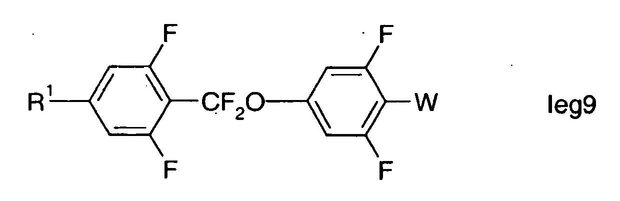

Die bevorzugten Verbindungen der Teilformel If umfassen diejenigen der Teilformeln Ifa bis Ife:

R¹-Phe-CH₂CH₂-A¹-Z¹-W Ifa

R¹-A¹-COO-Phe-Z¹-W Ifb

R¹-Cyc-Z¹-Cyc-Z¹-W Ifc

R¹-Phe-Z¹-Phe-Z¹-W Ifd

R¹-Cyc-CH₂CH₂-Phe-Z¹-W Ife

Die bevorzugten Verbindungen der Teilformeln Ig bis In umfassen diejenigen der Teilformeln Iga bis Ima:

R¹-A¹-Cyc-Cyc-W Iga

R¹-A¹-Cyc-Phe-W Igb

R¹-Phe-Phe-Phe-W Igc

R¹-Thp-Dio-Phe-W Igd

R¹-Dio-Thp-Phe-W Ige

R¹-A¹-CH₂CH₂-A¹-Phe-W Iha

R¹-Phe-Z¹-A¹-Phe-W Ihb

R¹-A¹-Phe-Z¹-Phe-W Iia

R¹-Cyc-Cyc-Phe-Z¹-W Ija

R¹-Cyc-Z¹-A¹-Z¹-Phe-W Ika

R¹-A¹-Z¹-Cyc-Phe-Z¹-W Ima

In den vorstehenden, bevorzugten Formeln Ia bis Ih und ihren Unterformeln besitzen R¹, A¹, Z¹ und W allgemein die oben angegebenen Bedeutungen.

In den vorstehenden, bevorzugten Formeln Ia bis Ih und ihren Unterformeln bedeutet R¹ vorzugsweise einen linearen Alkyl- bzw. Alkoxyrest mit 1 bis 7 C-Atomen oder einen linearen Alkenyl- bzw. Alkenyloxyrest mit 2 bis 7 C-Atomen und besonders bevorzugt einen linearen Alkylrest mit 1 bis 7 C-Atomen oder einen linearen Alkenylrest mit 2 bis 7 C-Atomen.

In den vorstehenden, bevorzugten Formeln Ia bis Ih und ihren Unterformeln bedeutet Z¹ jeweils unabhängig voneinander, gleich oder verschieden, vorzugsweise -CH₂CH₂-, -C=C-, -C≡C-, -CF₂CF₂-, -(CO)O-, -O(CO)- oder -CF₂O-, besonders bevorzugt -CF₂O- oder -CH₂CH₂-.

Falls R¹ in den vor- und nachstehenden Formeln einen Alkylrest bedeutet, so kann dieser geradkettig oder verzweigt sein. Besonders bevorzugt ist er geradkettig, hat 1, 2, 3, 4, 5, 6 oder 7 C-Atome und bedeutet demnach Methyl, Ethyl, Propyl, Butyl, Pentyl, Hexyl oder Heptyl, ferner Octyl, Nonyl, Decyl, Undecyl, Dodecyl, Tridecyl, Tetradecyl oder Pentadecyl.

Falls R¹ einen Alkylrest bedeutet, in dem eine CH₂-Gruppe durch -O-ersetzt ist, so kann dieser geradkettig oder verzweigt sein. Vorzugsweise ist er geradkettig und hat 1 bis 10 C-Atome. Besonders bevorzugt ist die erste CH₂-Gruppe dieses Alkylrestes durch -O- ersetzt, so dass der Rest R¹ die Bedeutung Alkoxy erhält und Methoxy, Ethoxy, Propoxy, Butoxy, Pentyloxy, Hexyloxy, Heptyloxy, Octyloxy oder Nonyloxy bedeutet.

Weiterhin kann auch eine CH₂-Gruppe an anderer Stelle durch -O- ersetzt sein, so dass der Rest R¹ und/oder R² vorzugsweise geradkettiges 2-Oxapropyl (= Methoxymethyl), 2- (= Ethoxymethyl) oder 3-Oxabutyl (= 2-Methoxyethyl); 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl bedeutet.

Falls R¹ einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Bevorzugte Alkenylgruppen sind C₂-C₇-1E-Alkenyl, C₄-C₇-3E-Alkenyl, C₅-C₇-4-Alkenyl, C₆-C₇-5-Alkenyl, und C₇-6-Alkenyl, besonders bevorzugt C₂-C₇-1E-Alkenyl, C₄-C₇-3E-Alkenyl und C₅-C₇-4-Alkenyl.

Beispiele besonders bevorzugter Alkenylgruppen sind Vinyl, 1 E-Propenyl, 1 E-Butenyl, 1E-Pentenyl, 1 E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl. Gruppen mit bis zu 5 Kohlenstoffatomen sind insbesondere bevorzugt.

Falls R¹ einen Alkylrest bedeutet, in dem eine CH₂-Gruppe durch -O- und eine durch -CO- ersetzt ist, so sind diese vorzugsweise benachbart. Somit beinhalten diese eine Acyloxygruppe -CO-O- oder eine Oxycarbonylgruppe -O-CO-. Besonders bevorzugt sind diese geradkettig und haben 2 bis 6 C-Atome.

Sie bedeuten demnach insbesondere Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl und 4-(Methoxycarbonyl)butyl.

Falls R¹ einen Alkylrest bedeutet, in dem eine CH₂-Gruppe durch unsubstituiertes oder substituiertes -CH=CH- und eine benachbarte CH₂-Gruppe durch -CO-, -CO-O- oder -O-CO- ersetzt ist, so kann dieser geradkettig oder verzweigt sein. Vorzugsweise ist er geradkettig und hat 4 bis 13 C-Atome. Er bedeutet demnach besonders bevorzugt Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl und 9-Methacryloyloxynonyl.

Falls R¹ einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R¹ einen mindestens einfach durch Halogen substituierten Alkyl- oder Alkenylrest bedeutet, so ist dieser Rest vorzugsweise geradkettig. Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I mit verzweigter Flügelgruppe R¹ können gelegentlich wegen einer besseren Löslichkeit in den üblichen, flüssigkristallinen Basismaterialien von Bedeutung sein, insbesondere aber als chirale Dotierstoffe, wenn sie optisch aktiv sind. Smektische Verbindungen dieser Art eignen sich als Komponente(n) für ferroelektrische Materialien.

Verzweigte Gruppen dieser Art enthalten vorzugsweise nicht mehr als eine Kettenverzweigung. Bevorzugte verzweigte Reste R¹ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentyloxy, 3-Methylpentyloxy, 2-Ethylhexyloxy, 1-Methylhexyloxy und 1-Methylheptyloxy.

Die Formel I und die Unterformeln umfassen, falls es sich um chirale Verbindungen handelt, üblicherweise die Racemate dieser Verbindungen, aber auch beide optisch reinen Komponenten für sich, sowie angereicherte Gemische dieser Komponenten.

Unter den Verbindungen der Formel I sowie den Unterformeln sind diejenigen bevorzugt, in denen mindestens eines der darin enthaltenen Elemente R¹, n, A¹ und Z¹ eine der angegebenen bevorzugten Bedeutungen hat. Besonders bevorzugte Verbindungen der Formel I ergeben sich aus entsprechend oder aus mehreren bevorzugten Elementen.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren, wobei des Heteroatom in der Regel bevorzugt näher an der Gruppe W lokalisiert ist.

Die Verbindungen der Formel I werden nach an sich bekannten Methoden dargestellt, wie sie in der Literatur (z. B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Ein weiterer Aspekt der Erfindung sind Verfahren zur Herstellung von Verbindungen der Formel I, die dadurch gekennzeichnet sind, dass es einen oder mehrere Verfahrenschritte umfasst, wodurch an ein 5,6-Dibrom- oder ein 5,6-Dihydroxy-Derivat des Indans oder des Benzo[1,3]dioxols in 5,6-Position ein Cyclopentanring angeschlossen wird. Der neu gebildete Cyclopentanring ist derart substituiert, dass sich unmittelbar oder nach einer weiteren Derivatisierung eine Substitution entsprechend den jeweiligen Verbindungen der Formel I ergibt. Weitere Details lassen sich aus einem odere mehreren der folgenden Syntheseschemas und den Erläuterungen entnehmen. Die speziellen angegebenen Substituenten, die nicht unmittelbar umgesetzt werden, können selbstverständlich analog der allgemeinen Formel I variiert werden, soweit sie keine chemischen Gruppen enthalten, die durch eine der angegebenen Reaktionen für den Fachmann offensichtlich beeinflusst werden könnte.

Eine Synthesevariante der erfindungsgemäßen Verbindungen, worin X jeweils eine Gruppe -CH₂- bedeutet und Y für CF³ steht, stellt sich wie folgt dar:

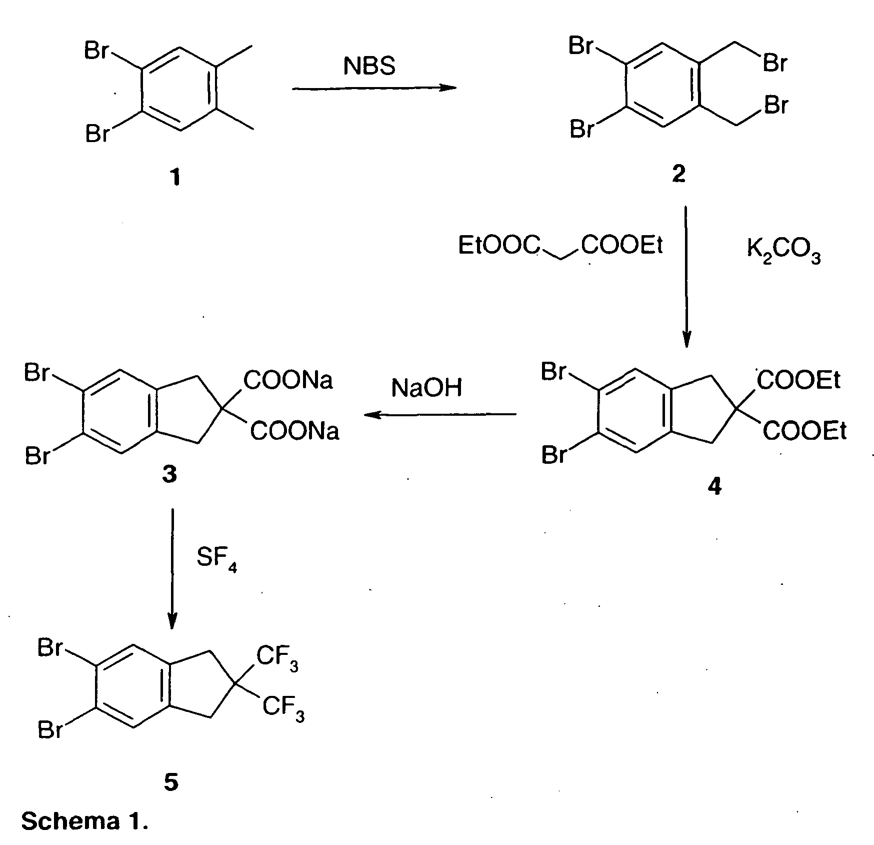

Aus der Verbindung der Formel 5 erhält man die entsprechenden erfindungsgemäßen Verbindungen analog dem experimentellen Beispiel 1. Dazu wird 5 gemäß Schema 2 umgesetzt.

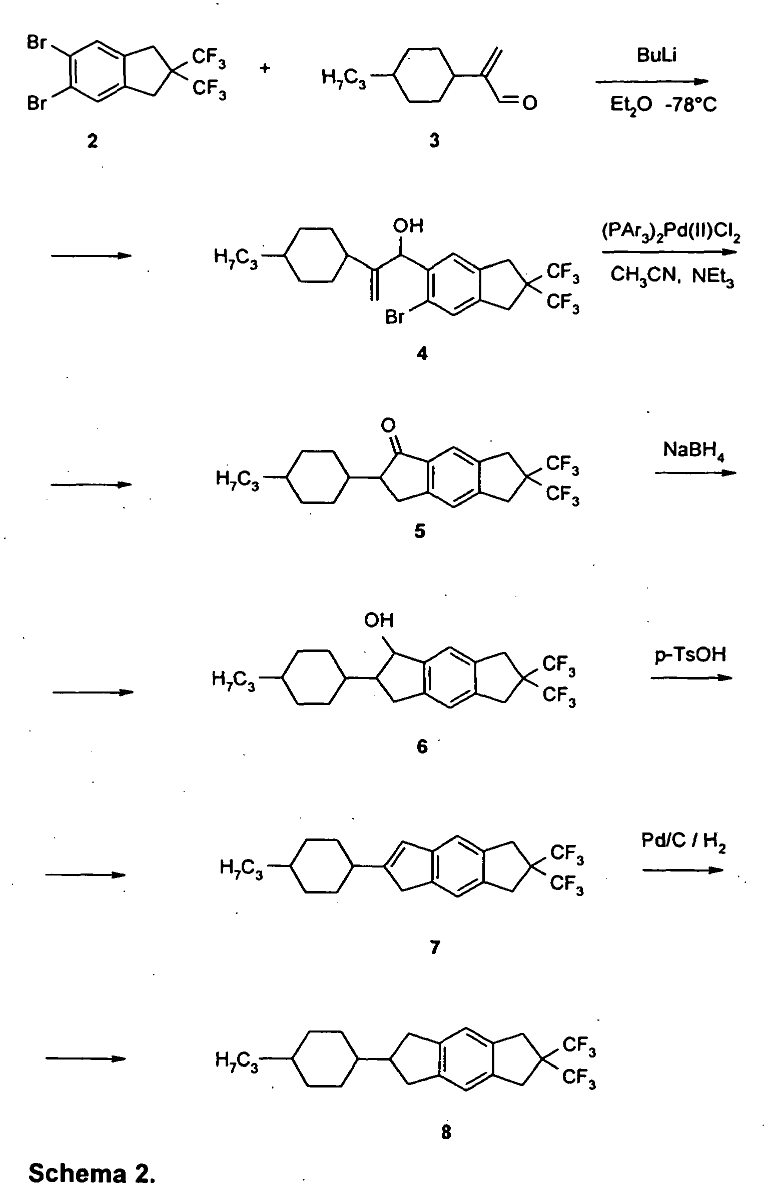

Die Ausgangsmaterialien für die hier oder in den Beispielen dargestellten Verfahren sind entweder bekannt oder können in Analogie zu bekannten Verbindungen hergestellt werden.

Die Dienodioxole werden analog den experimentellen Beispielen hergestellt, z. B. aus Indandiolen gemäß Schema 3.

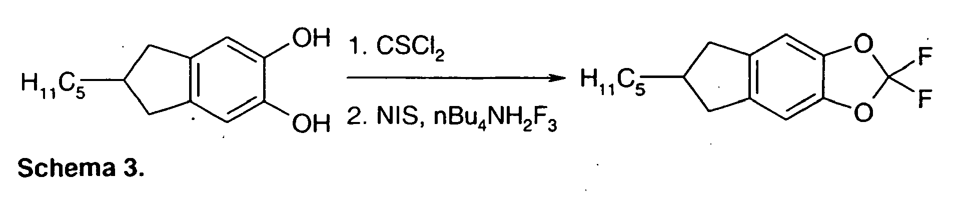

Eine Synthesevariante zur Herstelllung der Indandiole ergibt sich außerdem aus den in DE 4303634 offenbarten fluorierten Indanen durch Reaktion mit Natriummethylat (Schema 4).

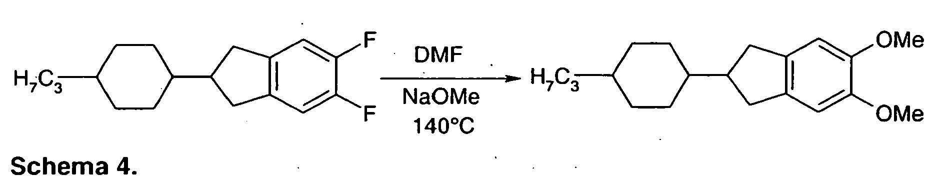

Gegebenenfalls können die Ausgangsstoffe auch in situ gebildet werden, derart, dass man sie aus dem Reaktionsgemisch nicht isoliert, sondern sofort weiter zu den Verbindungen der Formel I umsetzt.

Die dargestellten Reaktionen sind nur als beispielhaft aufzufassen. Der Fachmann kann entsprechende Varianten der vorgestellten Synthesen vornehmen sowie auch andere geeignete Synthesewege beschreiten, um die erfindungsgemäßen Verbindungen der Formel I zu erhalten.

Die Synthesen verschiedener erfindungsgemäßer 1,2,3,6,7,8-Hexahydro-s-indacen- und 6,7-Dihydro-5H-indeno[5,6-d][1,3]dioxol-Derivate der allgemeinen Formel I werden außerdem in den Beispielen ausführlich beschrieben. Die Synthesestrategie lässt sich durch die Wahl geeigneter Ausgangmaterialien auf die Synthese einer Vielfalt von erfindungsgemäßen Verbindungen anwenden. Beispielsweise können die vorhandenen Alkylketten durch verschiedene Ketten und Ringe gemäß der Formel I ersetzt werden. Ebenso können an der Reaktion unbeteiligte Ringe durch andere Ringe und Kombinationen aus Ketten und Ringen gemäß Formel I ersetzt werden.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile der erfindungsgemäßen Medien in Frage kommenden Verbindungen lassen sich durch die Formeln 1, 2, 3, 4 und 5 charakterisieren:

R'-L-E-R" 1

R'-L-(CO)O-E-R" 2

R'-L-O(CO)-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbildern gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, F, Cl, CN, NCS, -(O)ᵢCH₃₋ₖFₖ, wobei i 0 oder 1 und k 1, 2 oder 3 ist.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢ CH₃₋ₖ Fₖ, wobei i 0 oder 1 und k 1, 2 oder 3 sind. Die Verbindungen, in denen R" diese Bedeutung hat, werden mit den Teilformeln 1 b, 2b, 3b, 4b und 5b bezeichnet. Besonders bevorzugt sind solche Verbindungen der Teilformeln 1 b, 2b, 3b, 4b und 5b, in denen R" die Bedeutung F, Cl, NCS, CF₃, OCHF₂ oder OCF₃ hat.

In den Verbindungen der Teilformeln 1 b, 2b, 3b, 4b und 5b hat R' die bei den Verbindungen der Teilformeln 1a bis 5a angegebenen Bedeutungen und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" CN. Diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1 c, 2c, 3c, 4c und 5c hat R' die bei den Verbindungen der Teilformeln 1a bis 5a angegebenen Bedeutungen und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus den Gruppen A, B und/oder C. Die Massenanteile der Verbindungen aus diesen Gruppen in den erfindungsgemäßen Medien sind vorzugsweise:
- Gruppe A:: 0 bis 90 %, vorzugsweise 20 bis 90 %, besonders bevorzugt 30 bis 90 %;
- Gruppe B:: 0 bis 80 %, vorzugsweise 10 bis 80 %, besonders bevorzugt 10 bis 65 %;
- Gruppe C:: 0 bis 80 %, vorzugsweise 5 bis 80 %, besonders bevorzugt 5 bis 50 %;
wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die erfindungsgemäßen Medien enthalten vorzugsweise 1 bis 40 %, besonders bevorzugt 5 bis 30 %, der erfindungsgemäßen Verbindungen. Des weiteren bevorzugt sind Medien, enthaltend mehr als 40 %, besonders bevorzugt 45 bis 90 %, an erfindungsgemäßen Verbindungen.
Die Medien enthalten vorzugsweise drei, vier oder fünf erfindungsgemäße Verbindungen.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe und/oder chirale Dotierstoffe zugesetzt werden. Die einzelnen, zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen; n und m sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt, getrennt vom Akronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nN.F.F | CₙH₂ₙ₊₁ | CN | F | F |
| nF | CₙH₂ₙ₊₁ | F | H | H |
| nF.F | CₙH₂ₙ₊₁ | F | H | F |
| nF.F.F | CₙH₂ₙ₊₁ | F | F | F |
| nOF | OCₙH₂ₙ₊₁ | F | H | H |
| nCl | CₙH₂ₙ₊₁ | Cl | H | H |
| NCl.F | CₙH₂ₙ₊₁ | Cl | H | F |
| nCl.F.F | CₙH₂ₙ₊₁ | Cl | F | F |
| nCF₃ | CₙH₂ₙ₊₁ | CF₃ | H | H |
| nCF₃.F | CₙH₂ₙ₊₁ | CF₃ | H | F |
| nCF₃.F.F | CₙH₂ₙ₊₁ | CF₃ | F | F |
| nOCF₃ | CₙH₂ₙ₊₁ | OCF₃ | H | H |
| nOCF₃.F | CₙH₂ₙ₊₁ | OCF₃ | H | F |
| nOCF₃.F.F | CₙH₂ₙ₊₁ | OCF₃ | F | F |
| nOCF₂ | CₙH₂ₙ₊₁ | OCHF₂ | H | H |
| nOCF₂.F | CₙH₂ₙ₊₁ | OCHF₂ | H | F |
| nOCF₂.F.F | CₙH₂ₙ₊₁ | OCHF₂ | F | F |
| nS | CₙH₂ₙ₊₁ | NCS | H | H |
| nS.F | CₙH₂ₙ₊₁ | NCS | H | F |
| nS.F.F | CₙH₂ₙ₊₁ | NCS | F | F |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |

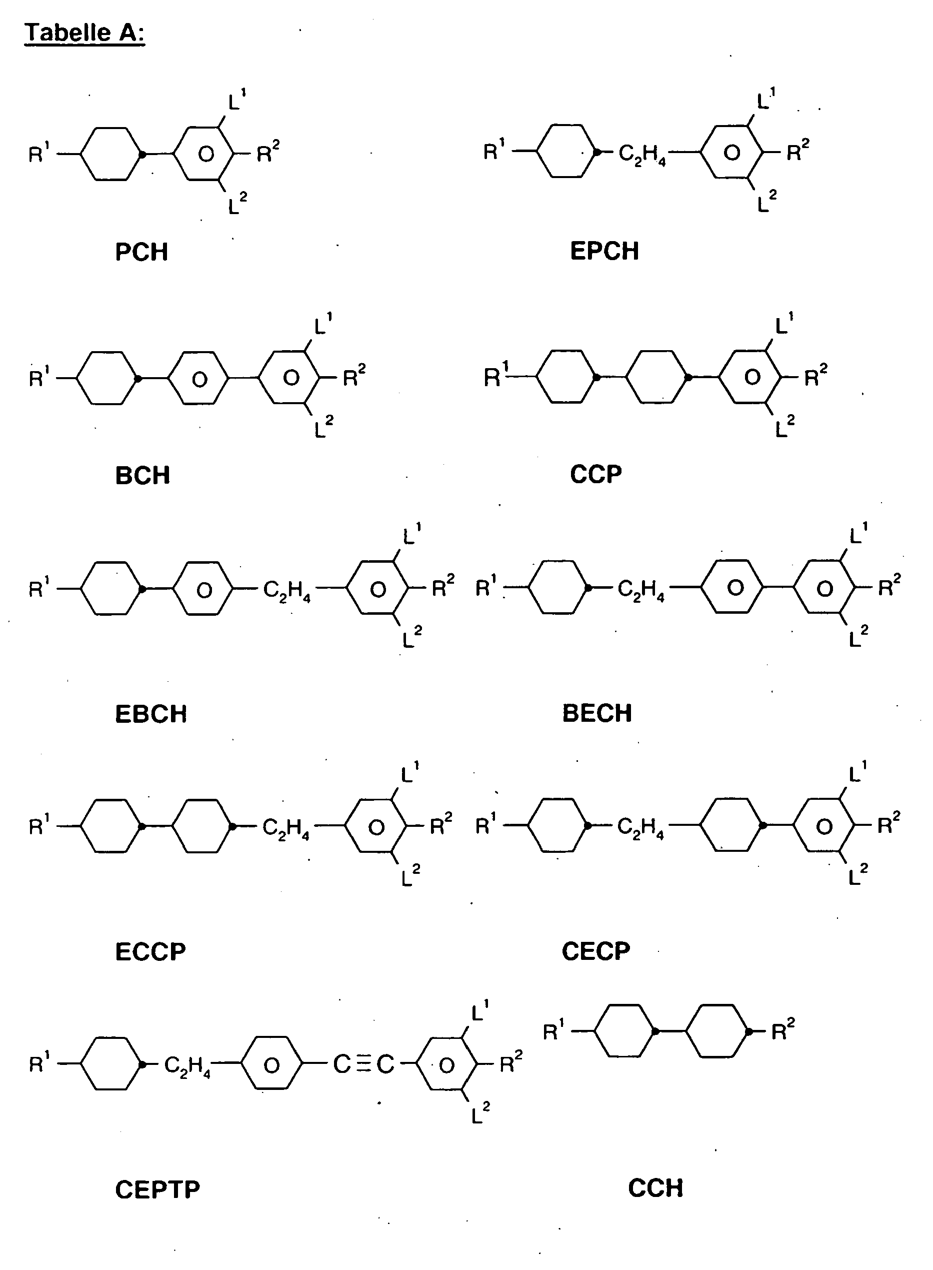

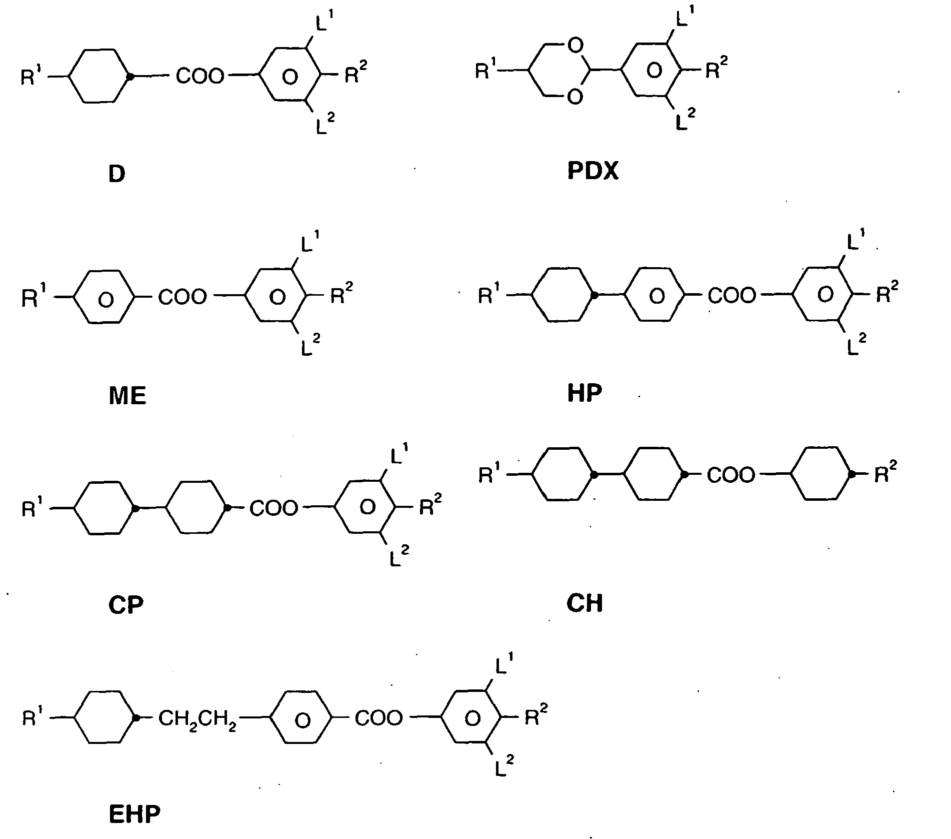

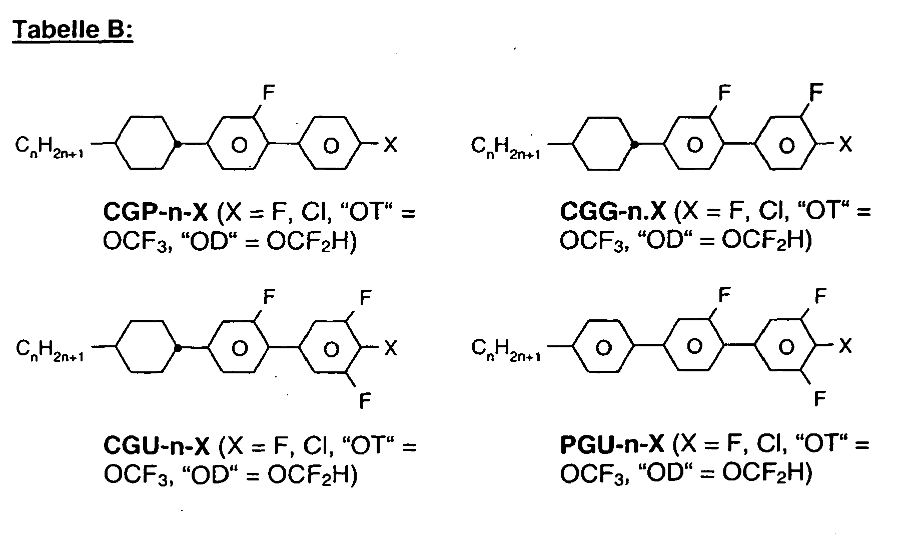

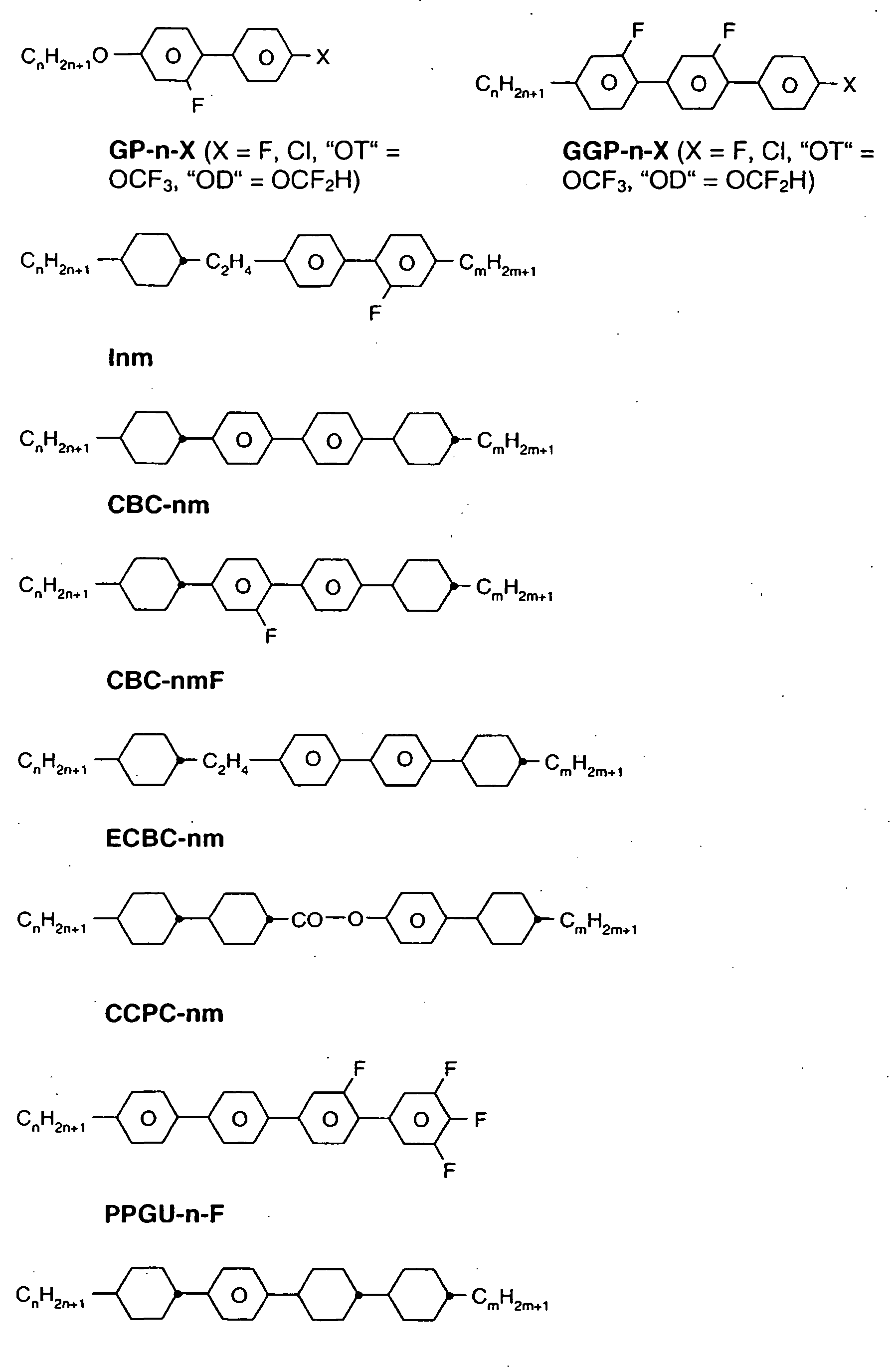

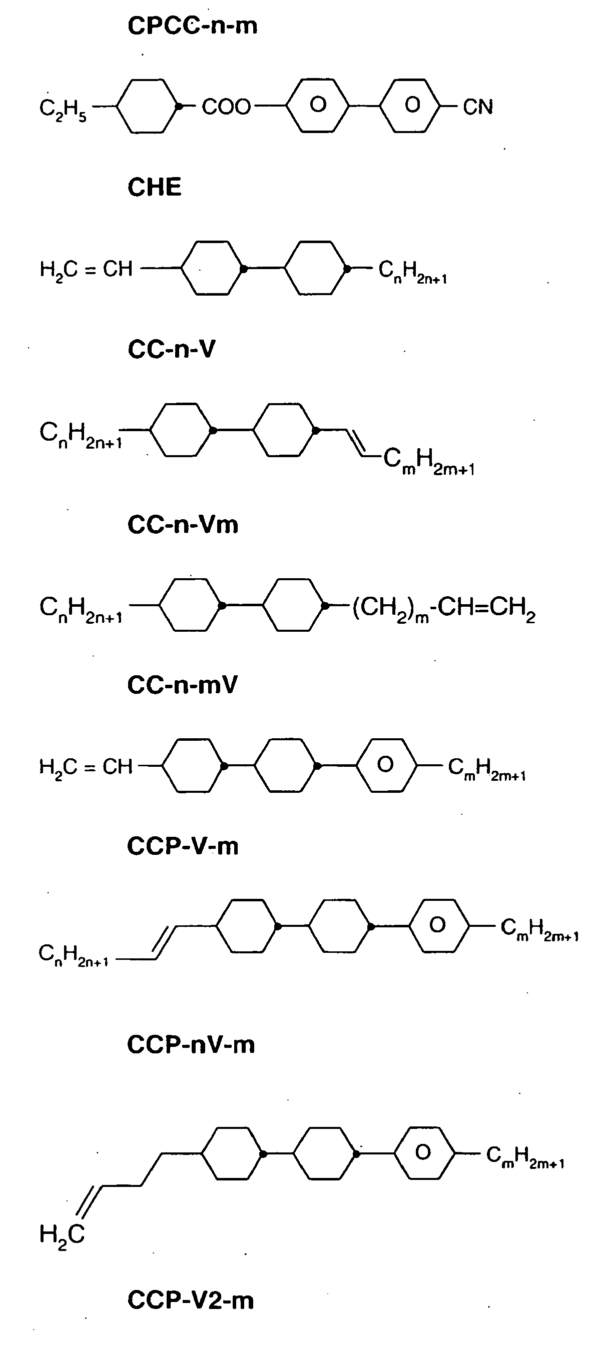

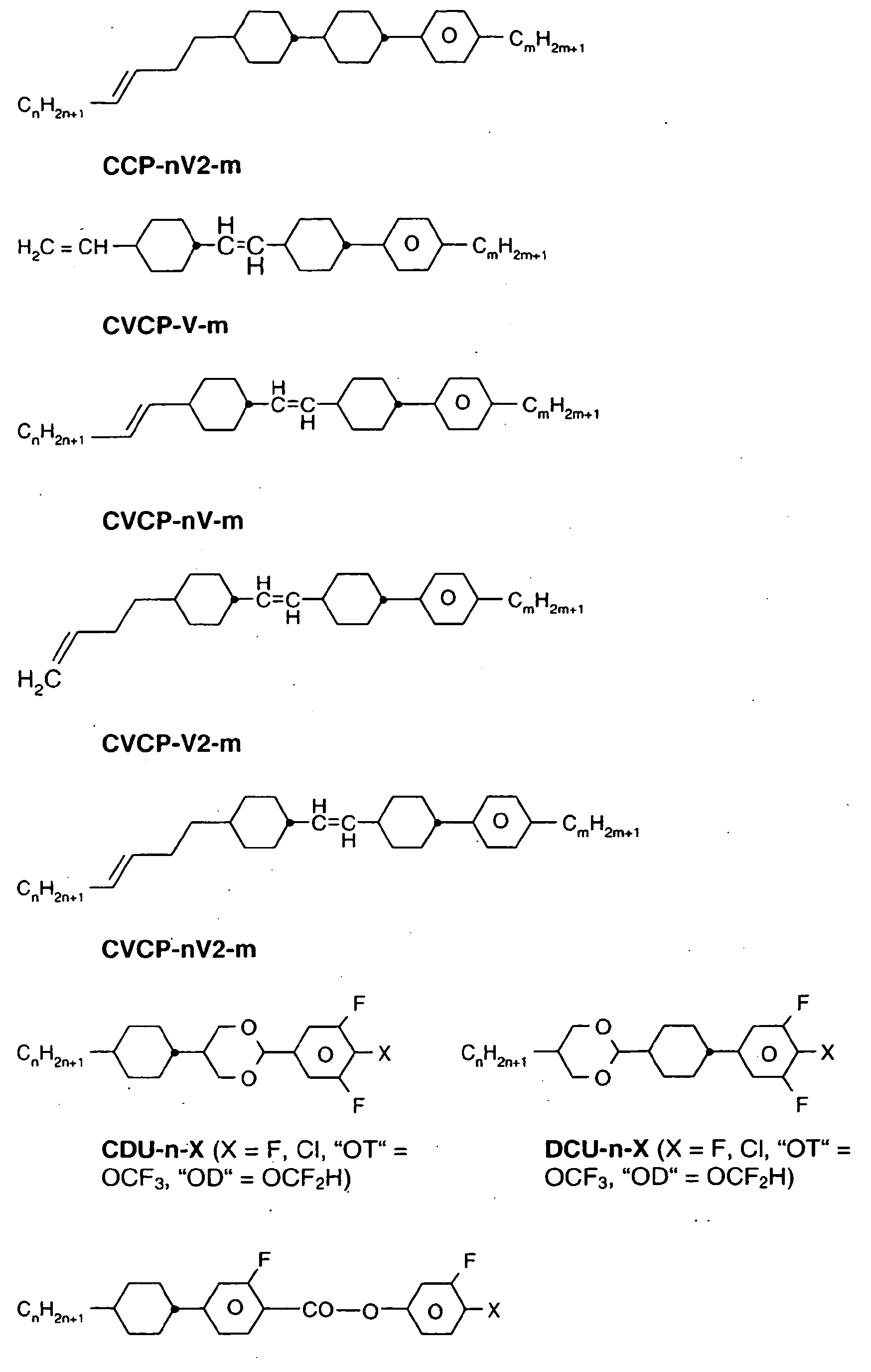

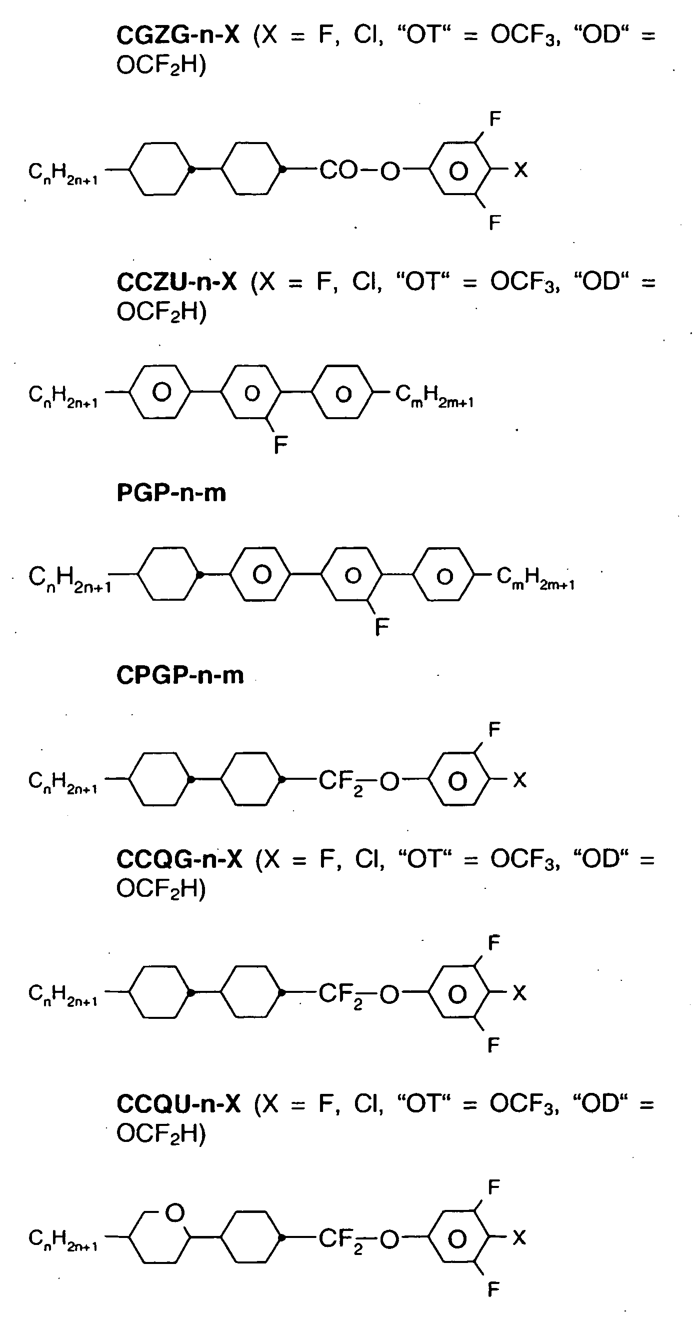

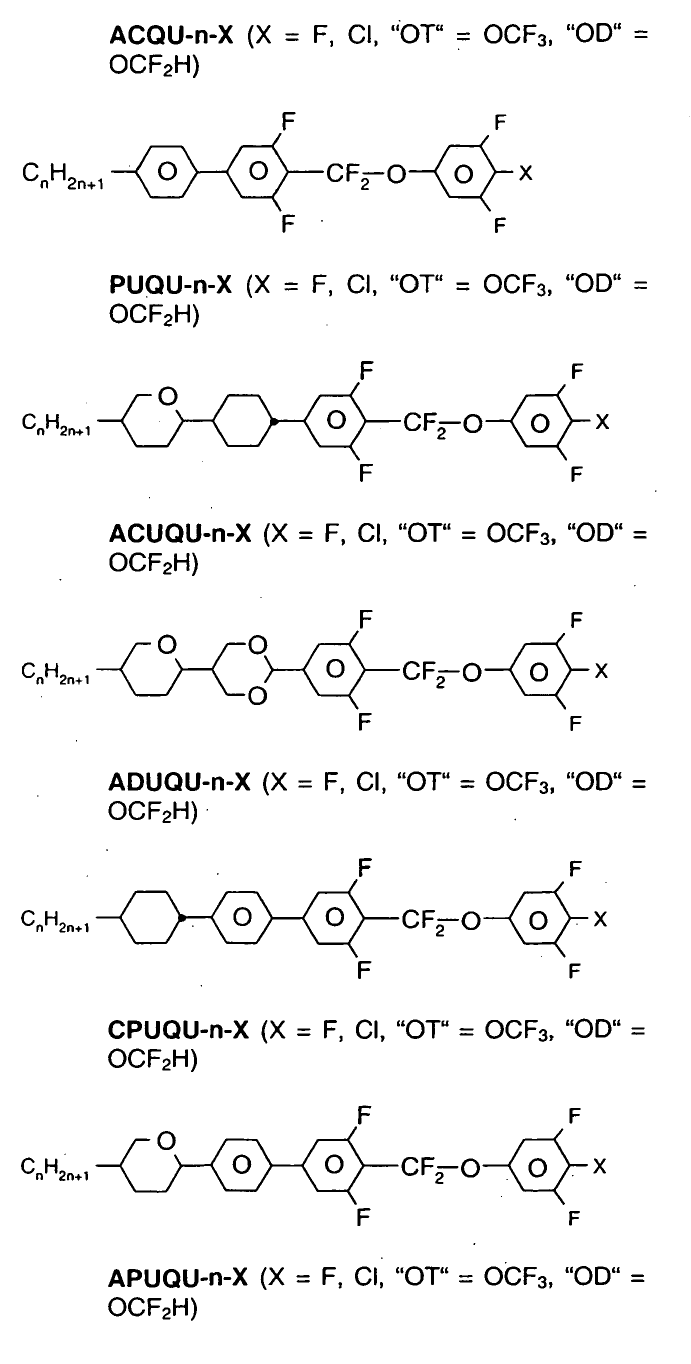

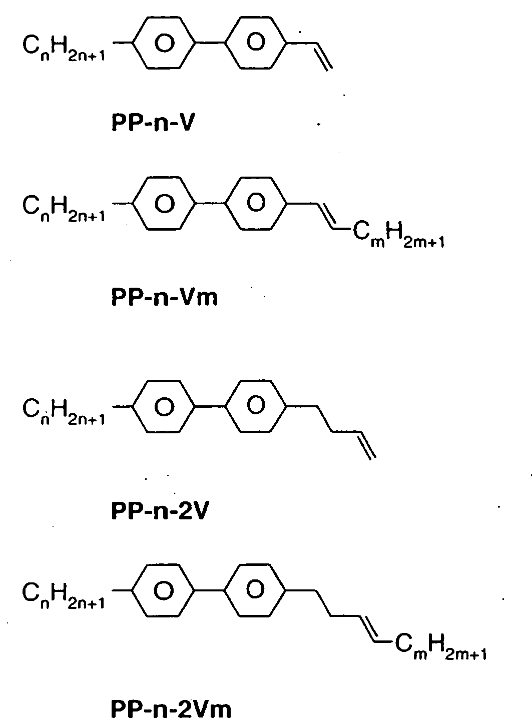

Die erfindungsgemäßen flüssigkristallinen Medien enthalten vorzugsweise:
- sieben oder mehr, bevorzugt acht oder mehr Verbindungen, bevorzugt von verschiedener Grundstruktur, ausgewählt aus der Gruppe von Verbindungen in den Tabellen A und B;
- eine oder mehrere, bevorzugt zwei oder mehrere, besonders bevorzugt drei oder mehrere Verbindungen, bevorzugt von verschiedener Grundstruktur, ausgewählt aus der Gruppe von Verbindungen in der Tabelle A;
- drei oder mehr, bevorzugt vier oder mehr Verbindungen, besonders bevorzugt fünf oder mehr Verbindungen, bevorzugt von verschiedener Grundstruktur, ausgewählt aus der Gruppe von Verbindungen in der Tabelle B.

**Tablelle C**

| |
|---|
| In der Tabelle C werden mögliche Dotierstoffe angegeben, die in der Regel den erfindungsgemäßen Mischungen zugesetzt werden. Vorzugsweise enthalten die Mischungen 0-10 Gew.%, insbesondere 0,01-5 Gew.% und besonders bevorzugt 0,01-3 Gew.% an Dotierstoffen. |
| 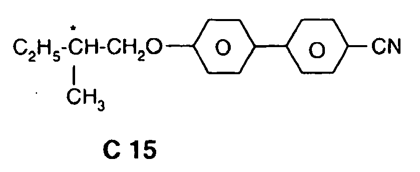 |
| 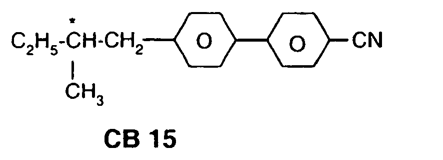 |
| 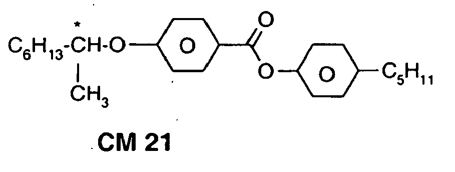 |
| 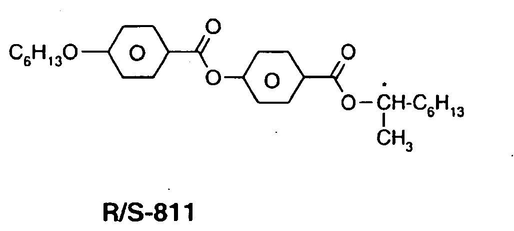 |
| 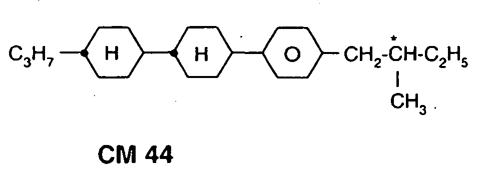 |
| 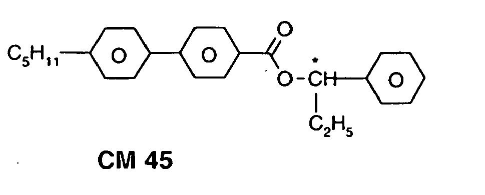 |
| 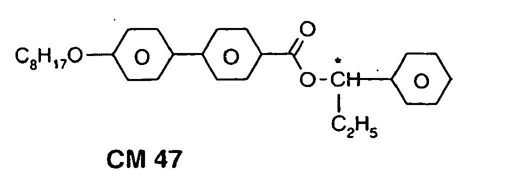 |
| 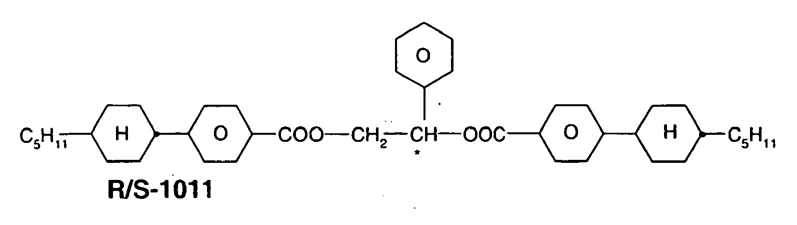 |
| 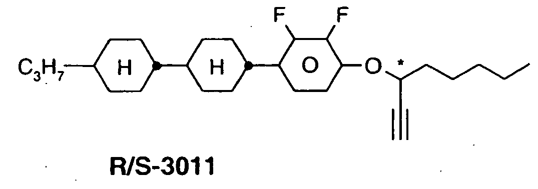 |
| 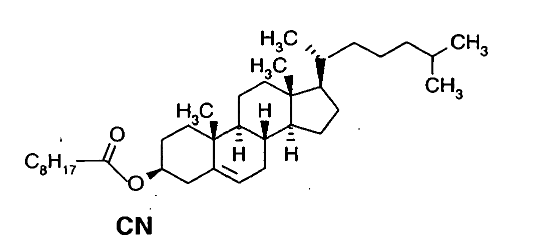 |
| 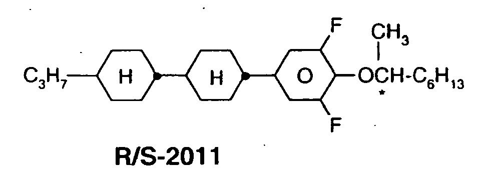 |
| 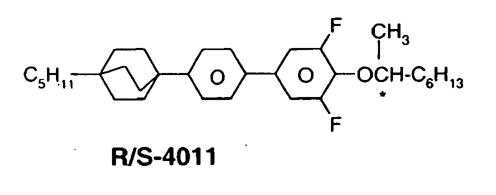 |
| 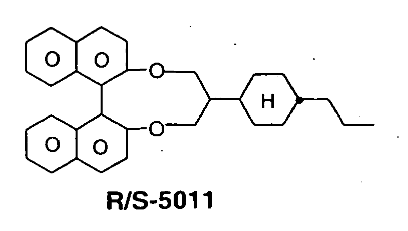 |

**Tabelle D**

| | |
|---|---|
| Stabilisatoren, die beispielsweise den erfindungsgemäßen Mischungen zugesetzt werden können, werden nachfolgend genannt. | |
| 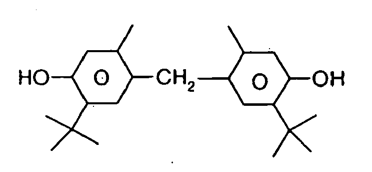 | 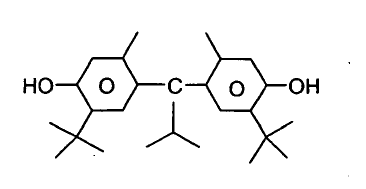 |
| 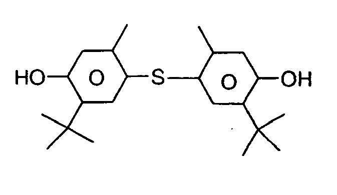 | 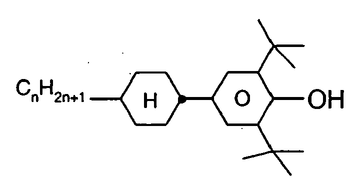 |
| 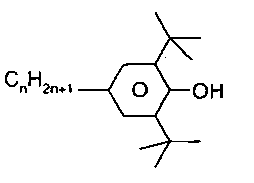 | 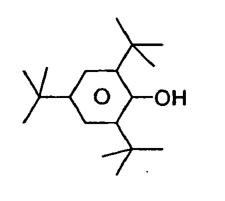 |
| 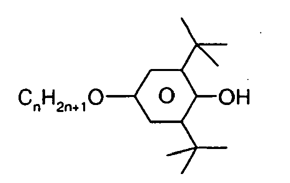 | 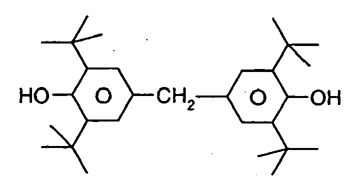 |
| 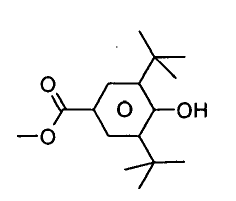 | 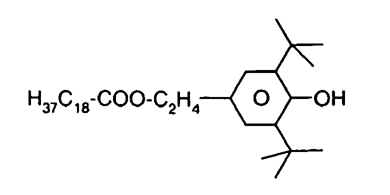 |
| 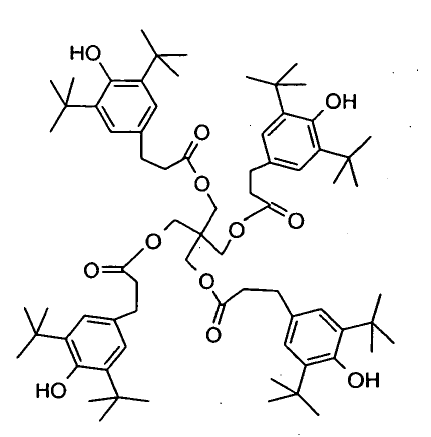 | 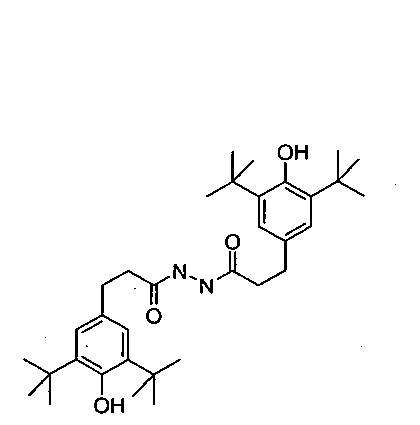 |
| 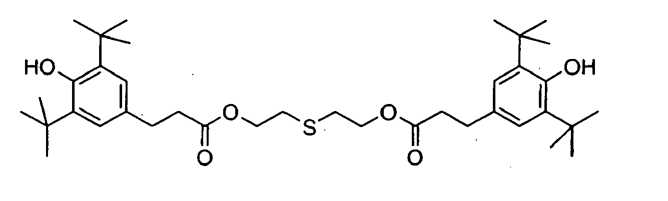 | |
| 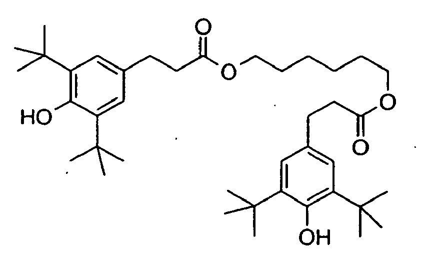 | 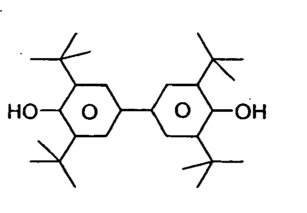 |
| 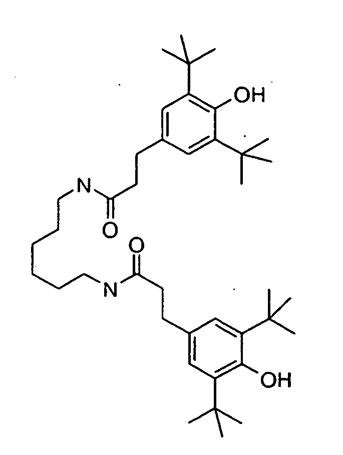 | 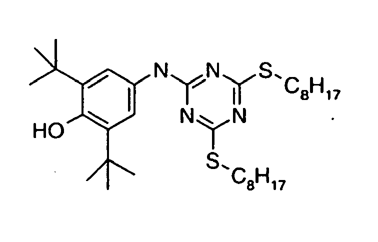 |
| 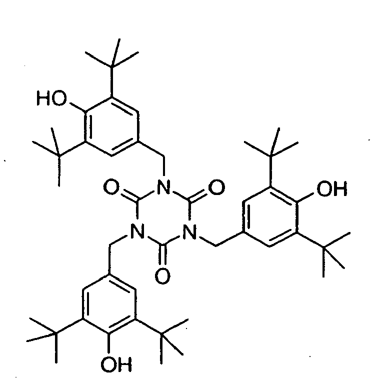 | 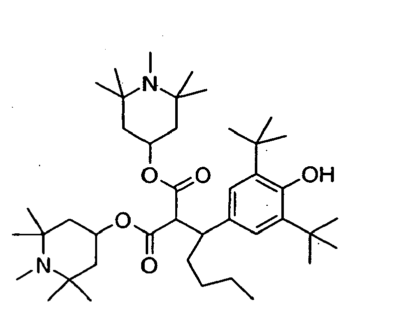 |
| 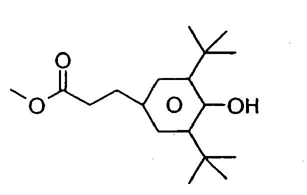 | 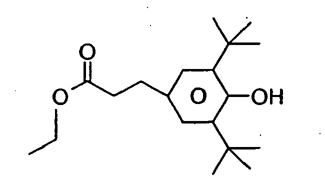 |
| 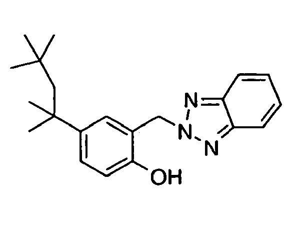 | 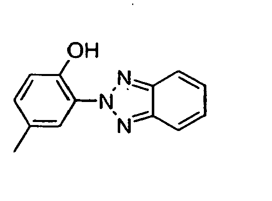 |
| 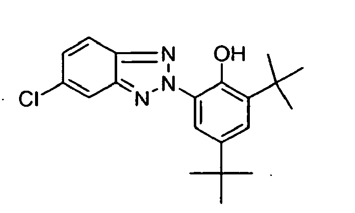 | 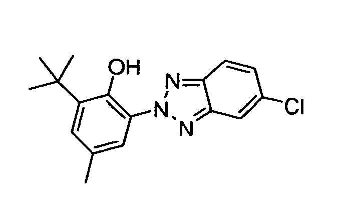 |
| 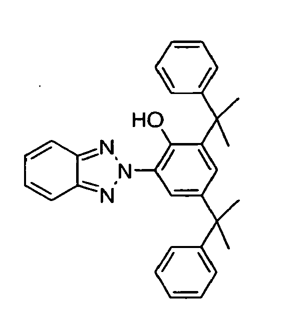 | 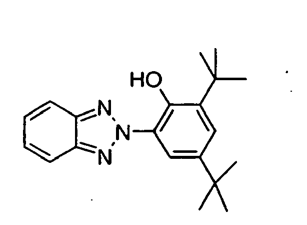 |
| 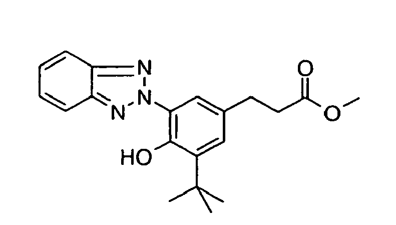 | 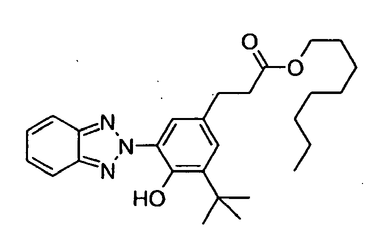 |
| 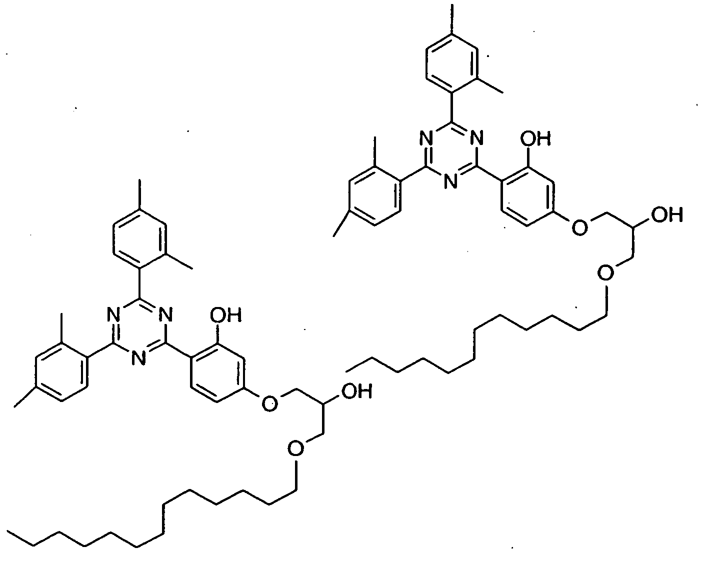 | |
| 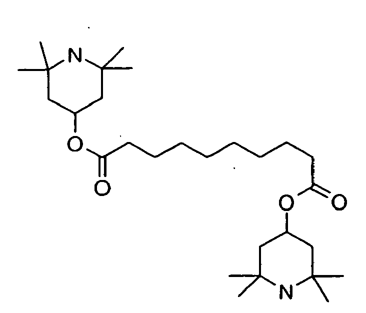 | 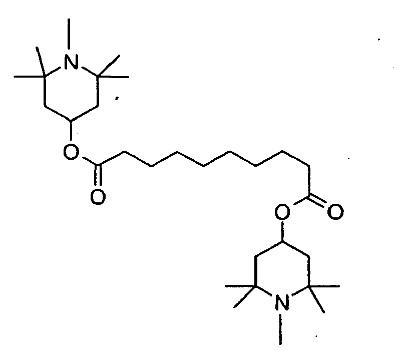 |
| 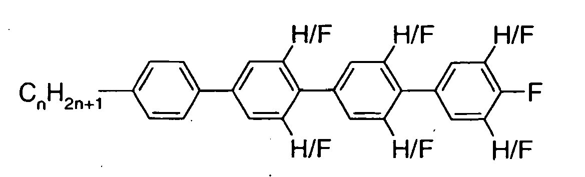 | |

Die folgenden Beispiele erläutern die Erfindung.

Vor- und nachstehend werden folgende Abkürzungen verwendet:
- RT: Raumtemperatur
- THF: Tetrahydrofuran
- MTB-Ether: Methyl-*tert*-butylether
- Py: Pyridin
- BuLi: n-Butyllithium
- DC: Dünnschichtchromatographie
- i. Vak.: unter vermindertem Druck
- DMF: Dimethylformamid
- NIS: N-Iodsuccinimid
- DMPU: N,N'-Dimethyl-N,N'-propylenharnstoff

### Beispiele

### Beispiel 1

Der Synthesebaustein **2** ist nach den Vorschriften von M. Kuroboshi, T. Hiyama Synlett (1994), 251-252 und E.L. Stogryn J. Org. Chrem. (1972), 37, 673 darstellbar. Die Herstellung des Ausgangsmaterials **3** ist nach F. Huet, M. Pellet, A. Lechevalier, J.-M. Conia, J. Chem. Res. Miniprint (1982), 9, 2528-2578 möglich.

### Reaktionsschritt 1.1

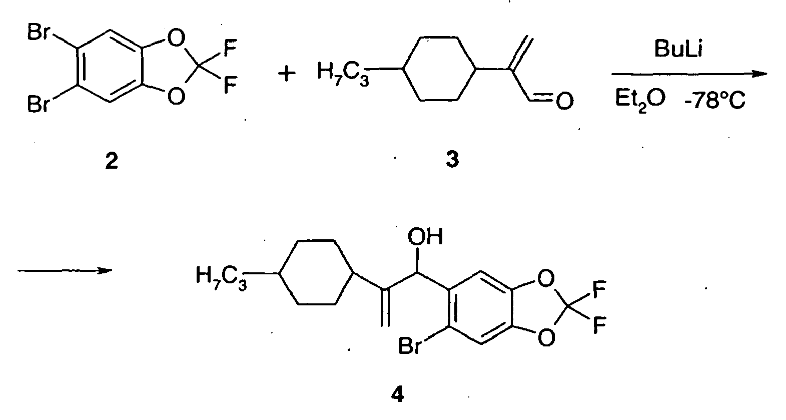

Unter Stickstoff wird 50,0 g (158 mmol) der Dibromphenylverbindung **2** in 200 ml Diethylether gelöst und bei -70°C mit 100 ml einer 15%igen Lösung von BuLi in n-Hexan versetzt und 1 h bei dieser Temperatur gehalten. Anschließend werden 29,0 g (161 mmol) des ungesättigten Aldehyds **3** dem Ansatz zugesetzt. Nach Rühren über Nacht bei RT wird der Ansatz hydrolysiert. Die wässerige Phase wird mit MTB-Ether extrahiert, die organische Phase wird über Natriumsulfat getrocknet, eingeengt und an Kieselgel gereinigt.

### Reaktionsschritt 1.2

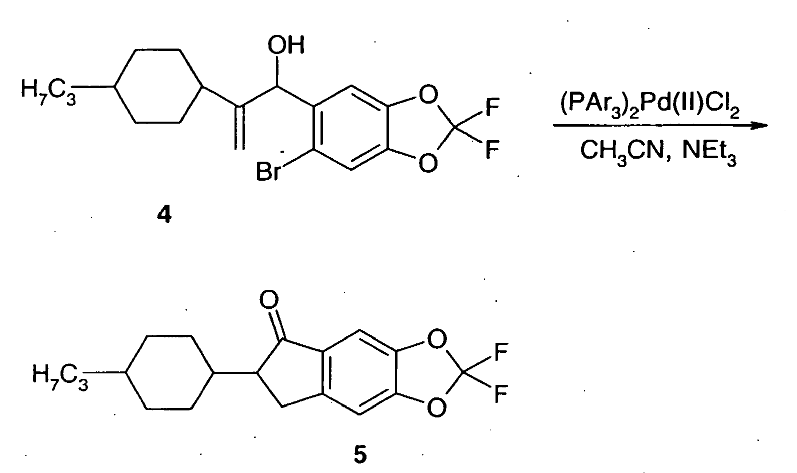

25,0 g (60,0 mmol) des Allylalkohols **4,** 4,3 g Bis(tri-o-tolylphosphin)-palladiumdichlorid und 25 ml Triethylamin werden in 180 ml Acetonitril gelöst und bis zur vollständigen Umsetzung des Allylalkohols 4 h unter Rückfluss erhitzt. Der erkaltete Ansatz wird auf Wasser gegeben, mit MTB-Ether extrahiert, über Natriumsulfat getrocknet, eingeengt und an Kieselgel gereinigt.

### Reaktionsschritt 1.3

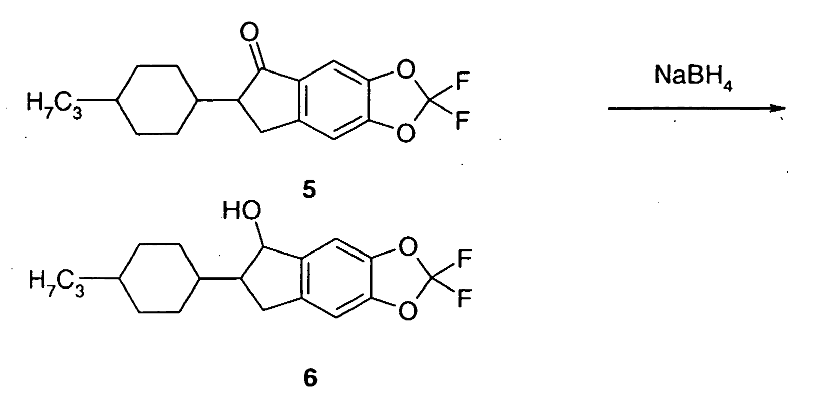

25,0 g (74,3 mmol) des Indanons **5** werden in 150 ml Ethanol gelöst und portionsweise mit 5,7 g (155 mmol) Natriumborhydrid versetzt. Nach Beendigung der Reaktion (DC) wird der Ansatz hydrolysiert, das Ethanol i.Vak. entfernt, der Rückstand in Wasser aufgenommen und mit MTB-Ether extrahiert. Nach dem Einengen wird das Produkt **6** ohne weitere Reinigung in der nächsten Stufe eingesetzt.

### Reaktionsschritt 1.4

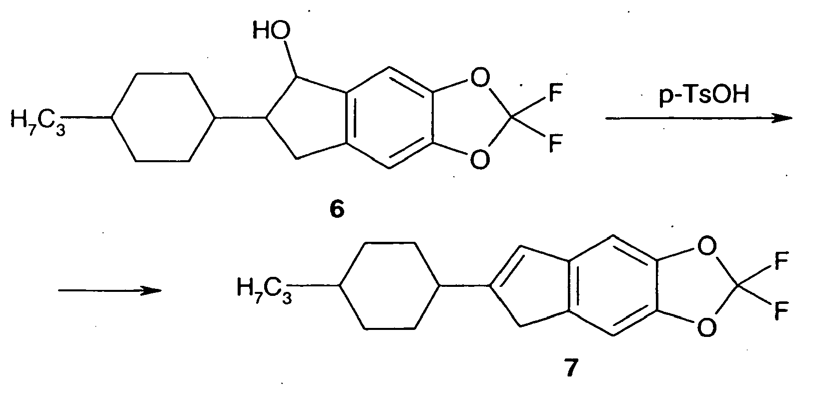

15,0 g (44,3 mmol) des Indanols **6** werden zusammen mit 500 mg *p-*Toluolsulfonsäure-Monohydrat in 150 ml Toluol gelöst und am Wasserabscheider unter Rückfluss erhitzt. Anschließend wird der Ansatz mit ges. Natriumhydrogencarbonatlösung gewaschen, über Natriumsulfat getrocknet eingeengt und an Kieselgel gereinigt.

### Reaktionsschritt 1.5

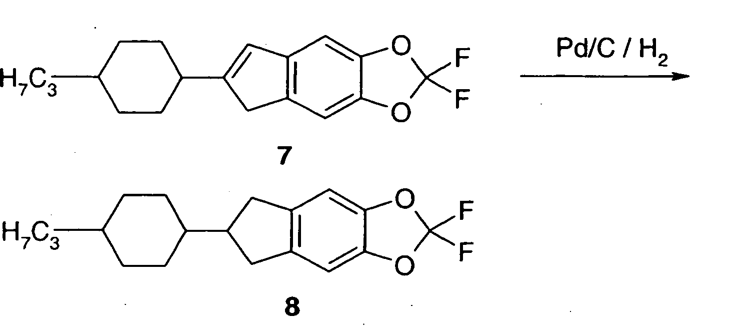

10,0 g (31,2 mmol) des Indens **7** werden in 50 ml THF gelöst und am Palladiumkatalysator (5% Pd/C) 9 h bei 10 bar Wasserstoffdruck bei RT hydriert. Anschließend wird der Katalysator abgetrennt, die Hydrierlösung eingeengt und der Rückstand an Kieselgel gereinigt.

### Beispiel 2

Die Darstellung des Indanderivats **10** aus dem entsprechendem Dibrombenzylbromid **9** wird analog WO 94/18285 vorgenommen.

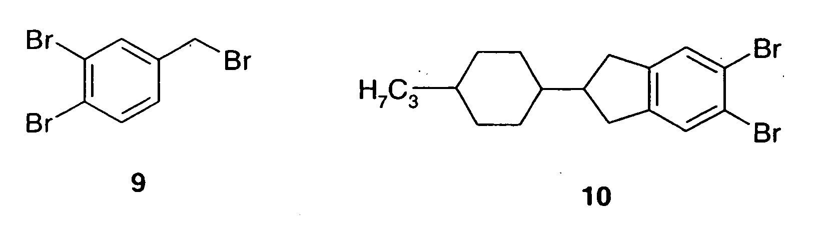

### Reaktionsschritt 2.1

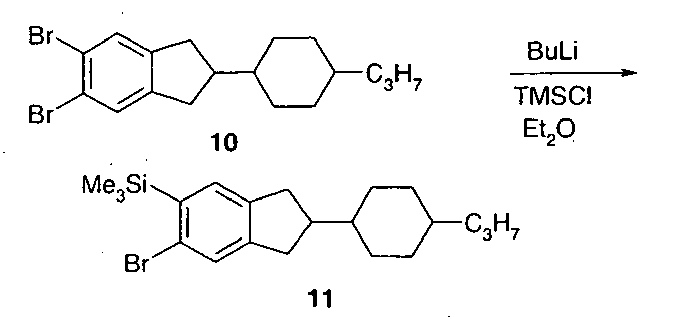

Unter Stickstoff werden 50,0 g (125 mmol) des Indans **10** in 200 ml Diethylether gelöst und bei -70°C mit 78,5 ml ein 15%igen Lösung von BuLi in n-Hexan versetzt und 1 h bei dieser Temperatur gehalten. Anschließend werden 16,5 ml (130 mmol) Trimethylsilylchlorid dem Ansatz zugesetzt. Nach Rühren über Nacht bei RT wird der Ansatz hydrolysiert. Die wässerige Phase wird mit MTB-Ether extrahiert, die organische Phase wird über Natriumsulfat getrocknet, eingeengt und an Kieselgel gereinigt.

### Reaktionsschritt 2.2

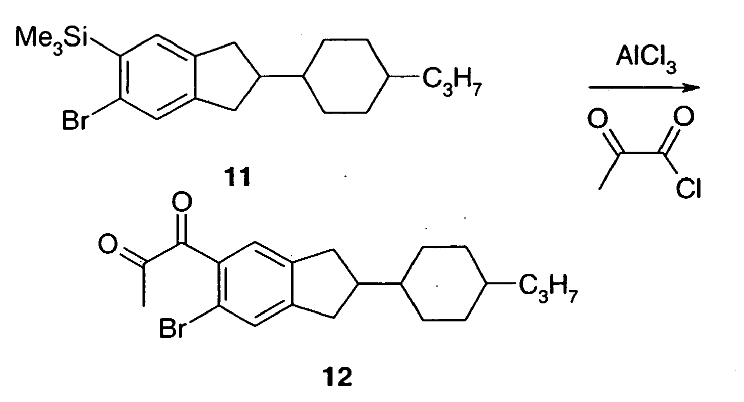

Unter Stickstoff wird 8,0 g (75,1 mmol) 2-Oxo-propionsäurechlorid in 40 ml Cyclohexan vorgelegt und bei 2-3°C mit einer Lösung von 30,0 g (76,2 mmol) der Silylverbindung **11** in 20 ml Cyclohexan versetzt. Anschließend werden bei -2 bis +2°C 10,7 g (80,0 mmol) Aluminiumchlorid portionsweise in den Ansatz eingetragen. Nach Entfernen der Kühlung erwärmt sich das Reaktionsgemisch auf 50°C und wird bis zum Abklingen der Reaktion bei dieser Temperatur gehalten. Der Ansatz wird auf Eiswasser gegeben und mit MTB-Ether extrahiert. Die organische Phase wird über Natriumsulfat getrocknet, eingeengt und an Kieselgel gereinigt.

### Reaktionsschritt 2.3

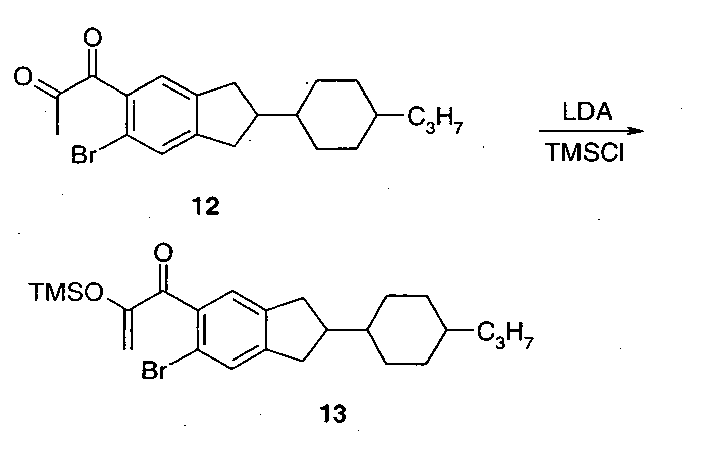

Unter Stickstoff werden 35,0 g (89,5 mmol) des Indans **12** in 150 ml THF gelöst und bei -70°C mit 46,4 ml ein 26%igen Lösung von Lithiumdiisopropylamid (LDA) in Cyclohexan/Ethylbenzol/THF versetzt und 1 h bei dieser Temperatur gehalten. Anschließend werden 12,0 ml (95,0 mmol) Trimethylsilylchlorid (TMSCl) dem Ansatz zugesetzt. Nach Rühren über Nacht bei RT wird der Ansatz hydrolysiert. Die wässerige Phase wird mit MTB-Ether extrahiert, die organische Phase wird über Natriumsulfat getrocknet, eingeengt und an Kieselgel gereinigt.

### Reaktionsschritt 2.4

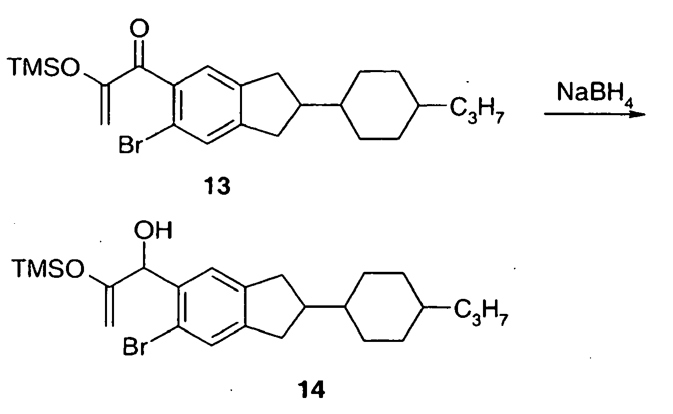

25,0 g (54,0 mmol) des Ketons **13** werden in 250 ml Ethanol gelöst und portionsweise mit 4,2 g (113 mmol) Natriumborhydrid versetzt. Nach Beendigung der Reaktion (DC) wird der Ansatz hydrolysiert, das Ethanol i.Vak. entfernt, der Rückstand in Wasser aufgenommen und mit MTB-Ether extrahiert. Nach dem Einengen wird das Produkt ohne weitere Reinigung in der nächsten Stufe eingesetzt.

### Reaktionsschritt 2.5

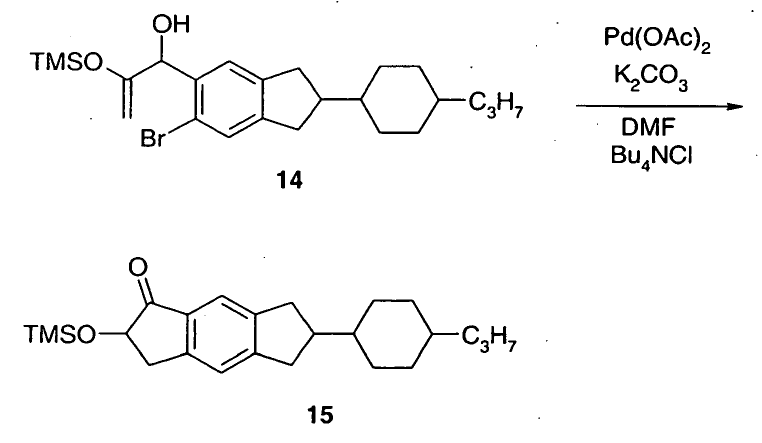

Unter Stickstoff wird eine Lösung von 17,0 g (36,5 mmol) des Silylenolethers **14** in 150 ml DMF mit 10,8 g (36,5 mmol) Tetrabutylammoniumchlorid-Monohydrat, 9,4 g (67,6 mmol) K₂CO₃ und 379 mg (1,7 mmol) Palladium(II)acetat versetzt und auf 80°C erwärmt. Nach Beendigung der Reaktion wird der abgekühlte Ansatz auf 1000 ml ges. Natriumchloridlösung gegeben und mit MTB-Ether extrahiert. Nach Trocknen über Natriumsulfat und Einengen der organischen Phase wird der erhaltene Rückstand an Kieselgel gereinigt.

### Reaktionsschritt 2.6

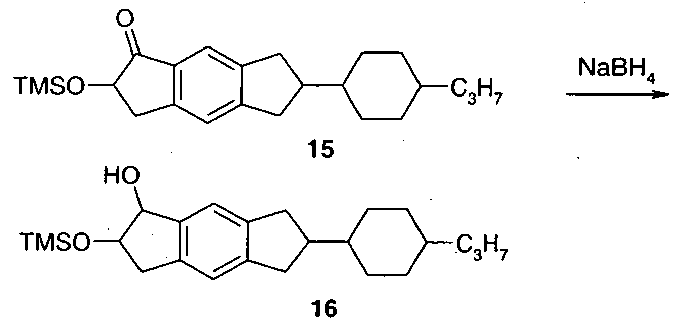

22,0 g (57,2 mmol) des Indanons **15** werden in 150 ml Ethanol gelöst und portionsweise mit 4,2 g (113 mmol) Natriumborhydrid versetzt. Nach Beendigung der Reaktion (DC) wird der Ansatz hydrolysiert, das Ethanol im Vakuum entfernt, der Rückstand in Wasser aufgenommen und mit MTB-Ether extrahiert. Nach dem Einengen wird das Produkt **16** ohne weitere Reinigung in der nächsten Stufe eingesetzt.

### Reaktionsschritt 2.7

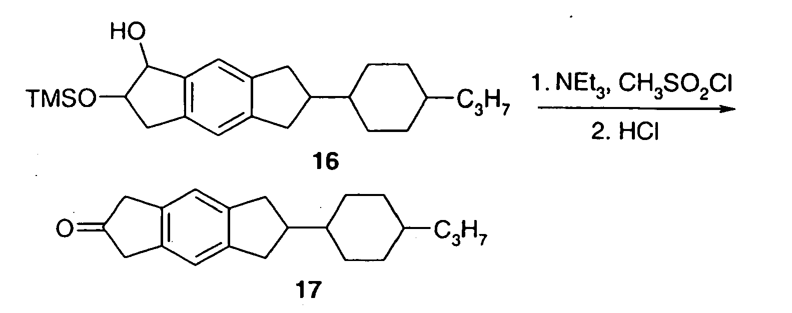

Unter Stickstoff werden 10,0 g (25,9 mmol) des Indanols, 16,8 ml Triethylamin und 60 mg 4-(Dimethylamino)-pyridin (DMAP) gelöst und bei 10-15°C mit 2,2 ml (28,4 mmol) Methansulfonsäurechlorid versetzt und anschließend 5 h unter Rückfluss erhitzt. Der abgekühlte Ansatz wird mit Wasser und ges. Natriumchloridlösung gewaschen und eingeengt. Der erhaltene Rückstand wird in THF aufgenommen und mit 1 ml konz. Salzsäure versetzt. Nach 3 h wird das Reaktionsgemisch auf ges. Natriumchloridlösung gegeben und mit MTB-Ether extrahiert. Die organische Phase wird eingeengt und an Kieselgel gereinigt.

### Reaktionsschritt 2.8

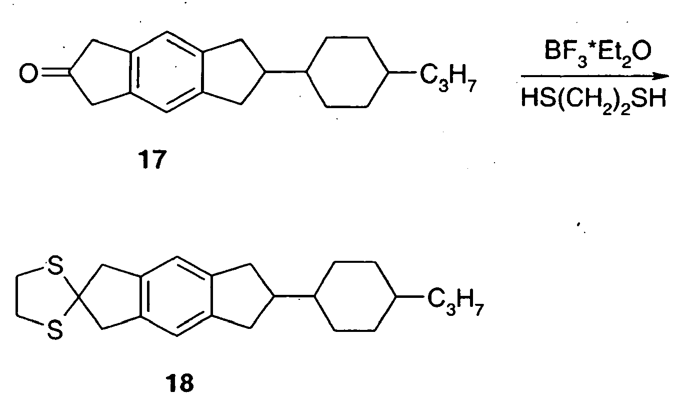

Unter Stickstoff wird eine Lösung von 8,0 g (27,0 mmol) des Indanons **17** und 5,5 ml (65,6 mmol) Ethandithiol in 65 ml Dichlormethan bei -15 bis -10°C mit 19 ml Bortrtifluorid-Diethylether-Komplex versetzt. Der Ansatz taut über Nacht auf und wird in ges. Natriumhydrogencarbonatlösung gegeben und bis zum Ende der Gasentwicklung gerührt. Die wässrige Phase wird mit DCM extrahiert und eingeengt. Der Rückstand wird an Kieselgel gereinigt.

### Reaktionsschritt 2.9

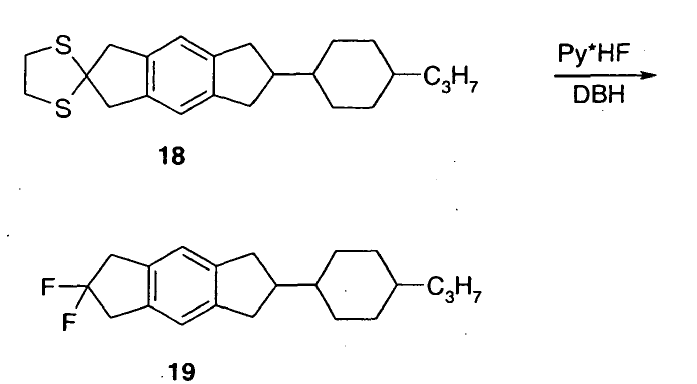

Eine Lösung von 8,0 g (21,5 mmol) des Dithiolans **18** in 30 ml Dichlormethan wird bei -75 °C in eine Suspension von 25,1 g (86,9 mmol) 1,3-Dibrom-5,5-dimethylhydantoin (DBH) in 65 ml Dichlormethan und 25 ml einer 65%igen Lösung von Fluorwasserstoff in Pyridin gegeben. Der Ansatz wird nach 3 h langsam auf 0°C erwärmt und in 750 ml einer eisgekühlten 2 N Natronlauge gegeben, die mit 60 ml einer 39%igen Natriumhydrogensulfitlösung versetzt worden war. Der pH-Wert wird auf pH 8 eingestellt und die wässrige Phase mit Methylenchlorid extrahiert. Die organische Phase wird über Natriumsulfat getrocknet, eingeengt und an Kieselgel gereinigt.

### Beispiel 3

### Reaktionsschritt 3.1

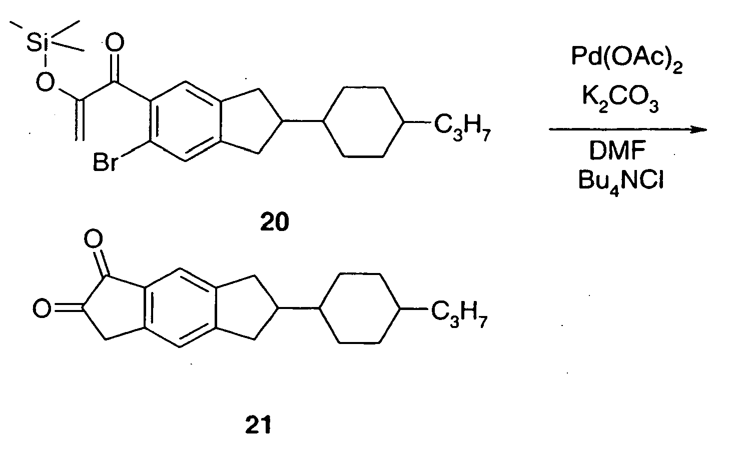

Unter Stickstoff wird eine Lösung von 15,0 g (32,4 mmol) des Silylenolethers **20** in 150 ml DMF mit 9,6 g (32,4 mmol) Tetrabutylammoniumchlorid-Monohydrat, 8,3 g (60,0 mmol) K₂CO₃ und 336 mg (1,5 mmol) Palladium(II)acetat versetzt und auf 80°C erwärmt. Nach Beendigung der Reaktion wird der abgekühlte Ansatz auf 1000 ml ges. Natriumchloridlösung gegeben und mit MTB-Ether extrahiert. Die organische Phase wird eingeengt, der erhaltene Rückstand in THF aufgenommen und mit 1 ml konz. Salzsäure versetzt. Nach 2 h wird der Ansatz auf ges. Natriumchloridlösung gegeben und mit MTB-Ether extrahiert. Nach Trocknen über Natriumsulfat und Einengen der organischen Phase wird der erhaltene Rückstand an Kieselgel gereinigt.

### Reaktionsschritt 3.2

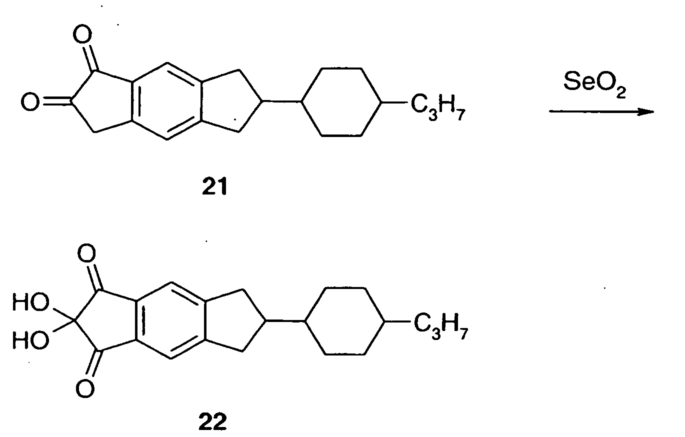

10,0 g (90,2 mmol) Selendioxid wird unter Erwärmen in 250 ml eines Dioxan/Wassergemisches (240:10) gelöst. Die abgekühlte Lösung wird mit 13,0 g (41,9 mmol) des Diketons 21 in Dioxan versetzt und über Nacht zum Sieden erhitzt. Das abgeschiedene Selen wird abgetrennt, und die Lösung auf Eiswasser gegeben. Nach Extraktion mit MTB-Ether wird die organische Phase über Natriumsulfat getrocknet und eingeengt. Der Rückstand wird an Kieselgel gereinigt.

### Reaktionsschritt 3.3

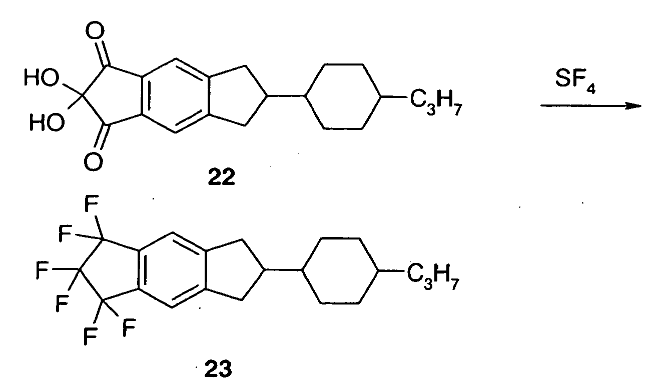

10,0 g (29,2 mmol) des Hydrats **22** werden mit 19,5 g (180 mmol) Schwefeltetrafluorid bei 120°C zur Reaktion gebracht. Das rohe Reaktionsprodukt wird in Dichlormethan aufgenommen, mit Wasser gewaschen, eingeengt und an Kieselgel gereinigt.

### Beispiel 4

Nach R. Mietchen, D. Rentsch, Tetrahedron (1992), 48(39), 8393-8400 wird das Ketal **24** von Hexafluoraceton hergestellt, aus dem analog Beispiel 1 die Verbindung **25** synthetisiert wird.

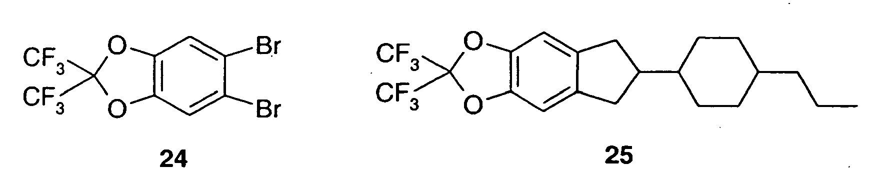

### Beispiel 5

### Reaktionsschritt 5.1

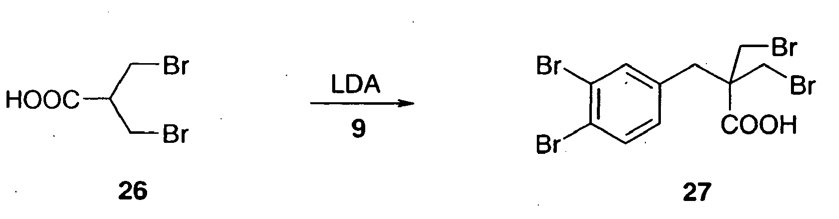

Unter Stickstoff wird eine Lösung von 30,0 g (122 mmol) der Carbonsäure **26** und 46,0 g (140 mmol) des Benzylbromids **9** (vgl. Beispiel 2) in 300 ml THF bei -70°C mit 129 ml (250 mmol) einer 26%igen Lösung von Lithiumdiisopropylamid in Cyclohexan/Ethylbenzol/THF versetzt und 3 h bei tiefer Temperatur gehalten. Nach Auftauen über Nacht wird der Ansatz auf Wasser gegeben und mit halbkonz. HCl angesäuert. Die wässrige Phase wird mit MTB-Ether extrahiert. Die organische Phase wird mit ges. Natriumchloridlösung gewaschen, über Natriumsulfat getrocknet und an Kieselgel gereinigt.

### Reaktionsschritt 5.2

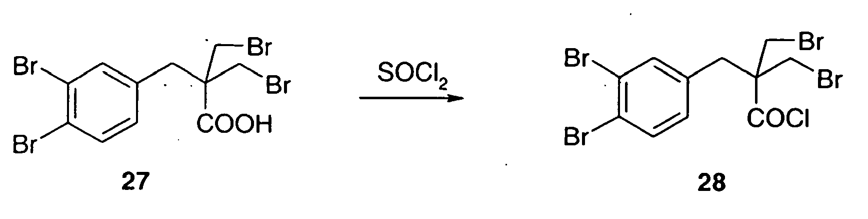

25,0 g der Carbonsäure 27 wird mit 10 ml (138 mmol) Thionylchlorid und einem Tropfen DMF versetzt. Nach Abklingen der Gasentwicklung wird der Ansatz 1 h auf 90°C erwärmt. Anschließend wird überschüssiges Thionylchlorid abdestilliert und der erhaltene Rückstand ohne weitere Reinigung in der Folgestufe eingesetzt.

### Reaktionsschritt 5.3

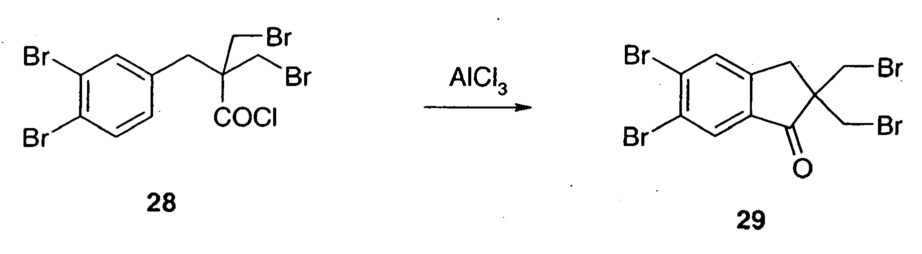

Unter Stickstoff wird eine Suspension von 6,1 g (45,6 mmol) Aluminiumchlorid in 50 ml Dichlormethan bei -20 °C langsam mit einer Lösung des Säurechlorids (23,0 g roh) in 25 ml Dichlormethan versetzt und 4 h bei dieser Temperatur gerührt. Die Hydrolyse des Ansatzes erfolgt mit Eis. Anschließend wird Wasser zugesetzt, bis eine Lösung entsteht. Die wässrige Phase wird mit Dichlomethan extrahiert, die organische Phase wird getrocknet, eingeengt und über Kieselgel gegeben.

### Reaktionsschritt 5.4

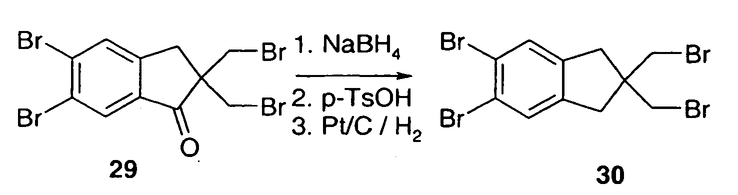

15,0 g (31,5 mmol) des Indanons **29** werden in 50 ml Ethanol gelöst und portionsweise mit 2,3 g (60,0 mmol) Natriumborhydrid versetzt. Nach Beendigung der Reaktion (DC) wird der Ansatz hydrolysiert, das Ethanol i.Vak. entfernt, der Rückstand in Wasser aufgenommen und mit MTB-Ether extrahiert. Nach dem Einengen wird das Produkt in 100 ml Toluol gelöst, mit p-Toluolsulfonsäure versetzt und am Wasserabscheider zum Sieden erhitzt. Der abgekühlte Ansatz wird mit ges. Natriumhydrogencarbonatlösung gewaschen und eingeengt. Der erhaltene Rückstand wird über eine dünne Schicht Kieselgel filtriert (Toluol). Das nun isolierte Rohmaterial wird in THF gelöst und am Platin-Katalysator hydriert. Die Hydrierlösung wird eingeengt und der Rückstand über Kieselgel gereinigt.

### Reaktionsschritt 5.5

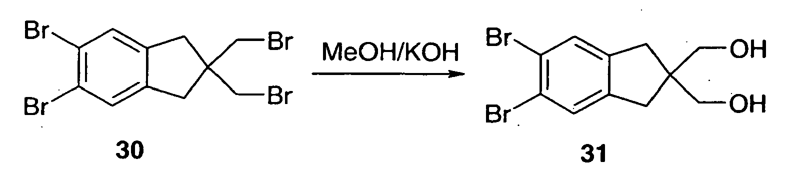

9,00 g (19,5 mmol) des Tetrabromids **30** wird mit 60 ml methanolischer Kaliumhydroxid-Lösung versetzt und zum Sieden erhitzt. Nach Beendigung der Reaktion (DC) wird das Methanol entfernt, der Rückstand mit Wasser verdünnt und angesäuert. Die wässerige Phase wird mit MTB-Ether extrahiert. Die organische Phase wird über Natriumsulfat getrocknet und eingeengt. Der erhaltene Rückstand wird an Kieselgel gereinigt.

### Reaktionsschritt 5.6

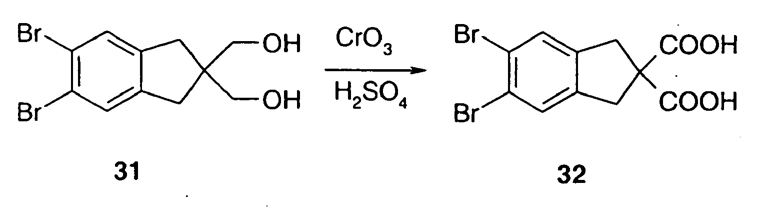

5,00 g (14,9 mmol) des Diols **31** wird in 25 ml Aceton gelöst und bei einer Temperatur von unter 30°C mit 8,1 ml Jones-Reagenz versetzt. Die während der Zugabe auftretende Braunfärbung des Ansatzes wird durch tropfenweise Zugabe konz. Schwefelsäure immer wieder rückgängig gemacht. Nach beendeter Zugabe wird die Kühlung entfernt und der Ansatz wird über Nacht bei Raumtemperatur gerührt. Der Ansatz wird auf 150 ml Wasser gegeben und mit MTB-Ether extrahiert. Die organische Phase wird mit ges. Natriumchloridlösung gewaschen, über Natriumsulfat getrocknet und eingeengt. Der Rückstand wird aus Isopropanol umkristallisiert.

### Reaktionsschritt 5.7

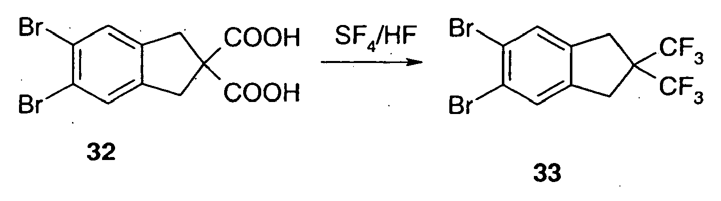

36,4 g (100 mmol) der Dicarbonsäure **32** wird bei 40°C mit 20,0 g (1,0 mol) Fluorwasserstoff und 70,0 g (650 mmol) Schwefeltetrafluorid 24 h zur Reaktion gebracht. Der Ansatz wird mit Kali-Lauge versetzt und mit n-Pentan extrahiert. Die organische Phase wird eingeengt und der erhaltene Rückstand wird an Kieselgel gereinigt.

Aus dem Indan **33** wird analog Beispiel 1 die Verbindung **34** hergestellt.

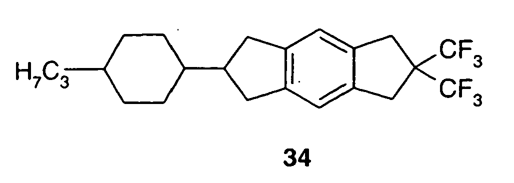

### Beispiel 6

### Reaktionsschritt 6.1

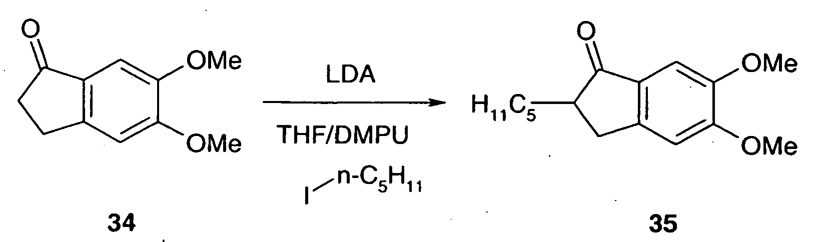

45 ml (90 mmol) einer 2 M-Lösung von Lithiumdiisopropylamid in THF/Ethylbenzol werden bei 0°C mit jeweils 100 ml THF und DMPU verdünnt und anschließend bei -70°C portionsweise mit einer Suspension von 17,5 g (90mmol) des Indanons **34** in THF versetzt. Nach 1 h werden 11,5 ml (90 mmol) lodpentan zugegeben. Der Ansatz wird 18 h bei RT gerührt, anschließend auf Wasser gegeben und angesäuert. Die wässerige Phase wird mit n-Heptan extrahiert. Die organische Phase wird mit ges. Hydrogencarbonatlösung gewaschen und eingeengt. Der Rückstand wird ohne weitere Reinigung in der Folgestufe eingesetzt.

### Reaktionsschritt 6.2

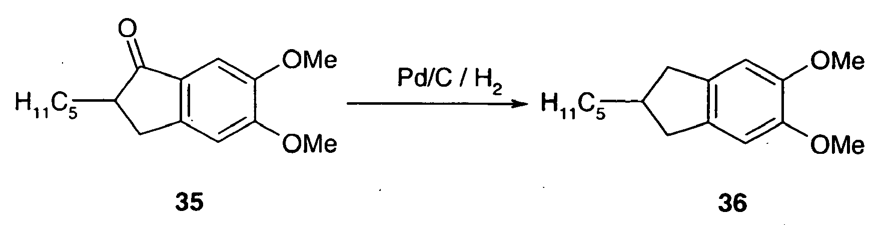

15,4 g (59 mmol) des Indanons **35** werden in 150 ml THF gelöst und bei RT am Palladiumkatalysator hydriert. Die Hydrierlösung wird eingeengt, und der Rückstand i.Vak. destilliert.

### Reaktionsschritt 6.3

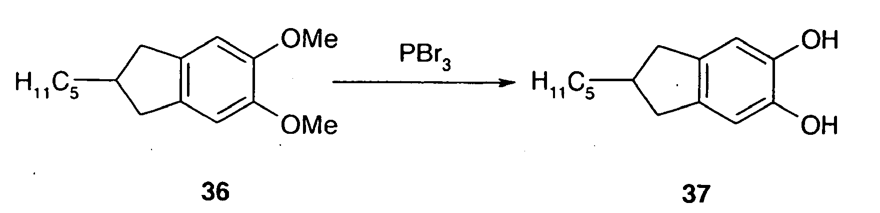

10 g (40 mmol) des Indans **36** werden unter Stickstoff in 260 ml Dichlormethan gelöst und bei RT mit 4,5 ml (47 mmol) Bortribromid versetzt. Nach 16 h bei RT wird der Ansatz auf 600 ml 1 M-Natronlauge gegeben. Anschließend wird die Reaktionsmischung mit Salzsäure angesäuert. Die organische Phase wird abgetrennt und eingeengt. Der Rückstand ist das gewünschte Indanderivat 37.

### Reaktionsschritt 6.4

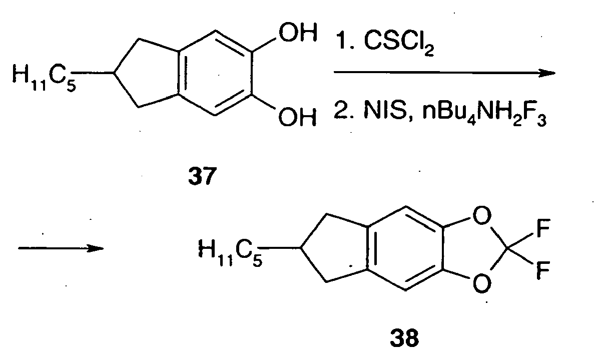

Die Synthese von **38** aus **37** erfolgt gemäß M. Kuroboshi, T. Hiyama Synlett (1994), 251-252.

## Patentansprüche

1. 1,2,3,6,7,8-Hexahydro-s-indacen- und 6,7-Dihydro-5H-indeno[5,6-d][1,3]dioxol-Derivate der allgemeinen Formel I 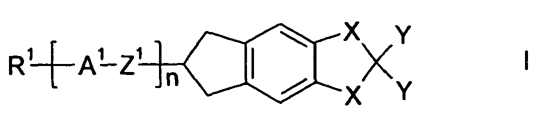 worin
R¹ H, Halogen, einen linearen oder verzweigten, unsubstituierten, einfach durch CN oder CF₃ substituierten oder mindestens einfach durch Halogen substituierten Alkylrest mit 1 bis 15 C-Atomen bedeutet, worin auch eine oder mehrere CH₂-Gruppen jeweils unabhängig voneinander durch -O-, -S-, -CO-, -(CO)O-, -CH=CH-, -CH=CF-, -CF=CF-oder -C≡C- so ersetzt sein können, dass Heteroatome nicht direkt miteinander verknüpft sind und unsymmetrische Gruppen in beiden Orientierungen vorliegen können,
A¹ jeweils unabhängig voneinander, gleich oder verschieden
a) trans-1,4-Cyclohexylen, worin auch eine oder mehrere nicht benachbarte CH₂-Gruppen durch -S- ersetzt sein können, Tetrahydropyran-2,5-diyl oder 1,3-Dioxan-2,5-diyl,
b) 1,4-Phenylen, worin eine oder zwei CH-Gruppen durch N ersetzt sein können und worin auch ein oder mehrere H-Atome gegen Halogen ersetzt sein können,
c) einen Rest aus der Gruppe 1,4-Bicyclo(2,2,2)-octylen, spiro[3.3]heptan-2,6-diyl, Cyclobutan-1,3-diyl, Piperidin-1,4-diyl, Naphthalin-2,6-diyl, Decahydronaphthalin-2,6-diyl oder 1,2,3,4-Tetrahydronaphthalin-2,6-diyl, und worin CH durch N ersetzt sein kann und worin auch ein oder mehrere H-Atome gegen Halogen ersetzt sein können, oder
d) 1,4-Cyclohexenylen,
X -CH₂-, -CF₂- oder -O-,
Y F, Cl, CF₃, CN, NCS, SCN, SF₅ oder 2- bis 6-C Perfluoralkyl,
Z¹ jeweils unabhängig voneinander, bei unsymmetrischen Gliedern Z¹ wahlweise in beiden Orientierungen vorliegend, eine Einfachbindung, -CH₂O-, -(CO)O-, -CF₂O-, -CF=CF-, -CH₂CH₂CF₂O- -CF₂CF₂-, -CH₂CF₂-, -CH₂CH₂-, -CH=CH-, -CH=CF- oder-C≡C-, und
n 0, 1, 2 oder 3
bedeuten.

2. Verbindungen gemäß Anspruch 1, **dadurch gekennzeichnet, dass** Y F oder CF₃ bedeutet.

3. Verbindungen gemäß Anspruch 1 oder 2, **dadurch gekennzeichnet, dass**
n gleich 0 ist.

4. Verbindungen gemäß Anspruch 1 oder 2, **dadurch gekennzeichnet, dass** n gleich 1 ist und es sich bei den Verbindungen der Formel I um Verbindungen der folgenden Formeln 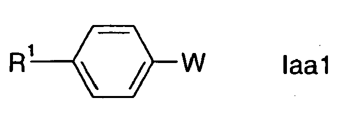 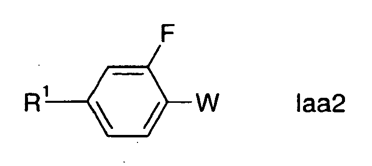 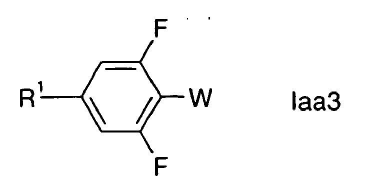 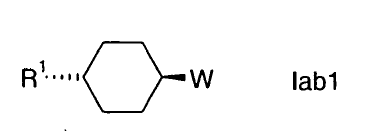 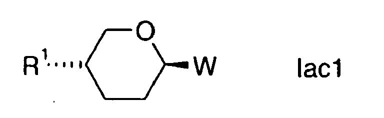 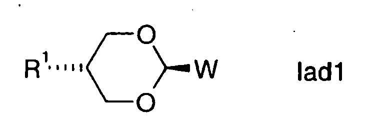 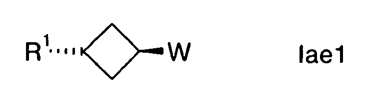 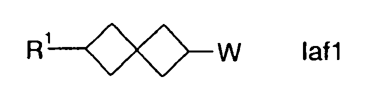 handelt, wobei W für den Molekülteil der Formel 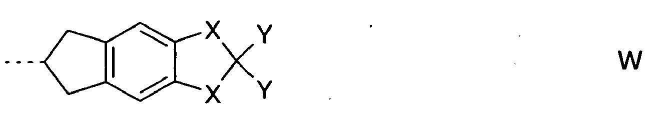 steht, worin X und Y wie in Anspruch 1 definiert sind.

5. Verbindungen gemäß Anspruch 1 oder 2, **dadurch gekennzeichnet, dass** es sich bei den Verbindungen der Formel I um Verbindungen mit zwei Ringen in der mesogenen Gruppe R¹-[A¹-Z¹]ₙ- handelt, ausgewählt aus der Gruppe der Verbindungen der Formeln Ic bis If
R¹-A¹-A¹-W Ic
R¹-A¹-A¹-Z¹-W Id
R¹-A¹-Z¹-A¹-W Ie
R¹-A¹-Z¹-A¹-Z¹-W If
wobei W wie in Anspruch 4 definiert ist.

6. Verbindungen gemäß Anspruch 5, **dadurch gekennzeichnet, dass** es sich bei den Verbindungen der Teilformel Ic um mindestens eine Verbindung ausgewählt aus den folgenden Formeln 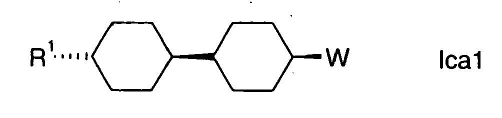 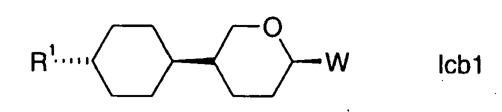 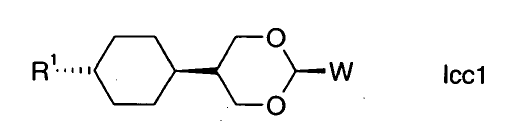 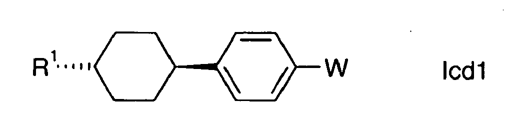 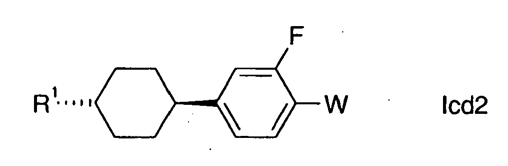 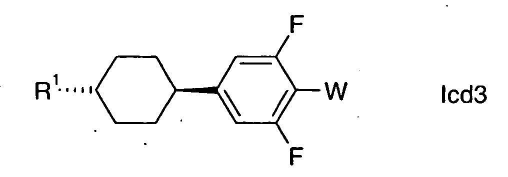 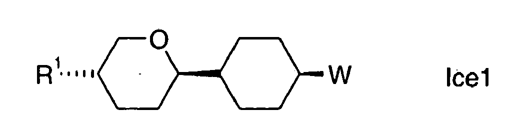 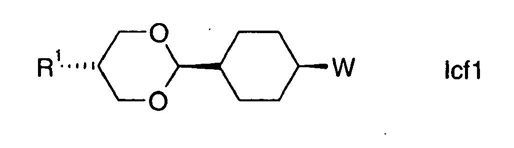 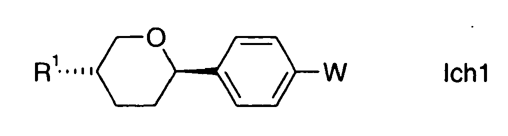 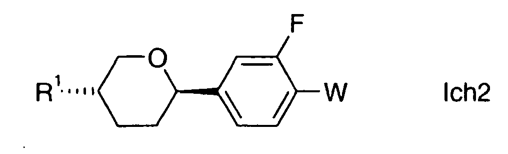 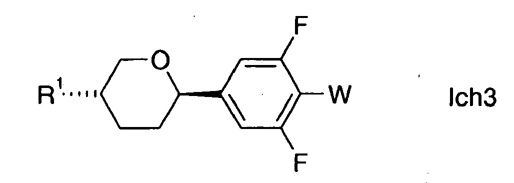 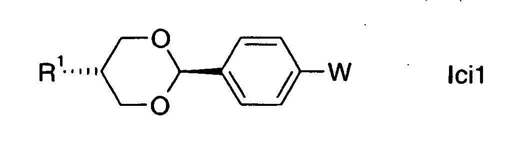 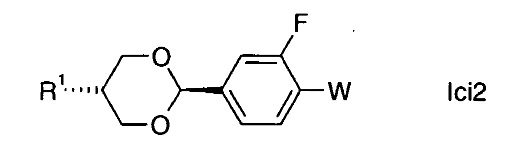 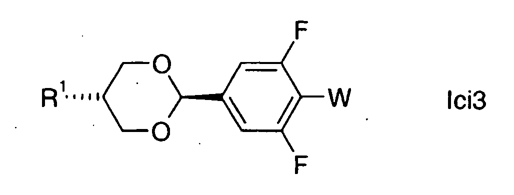 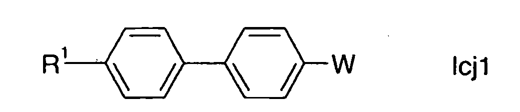 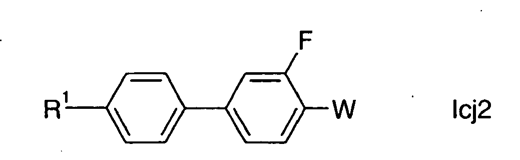 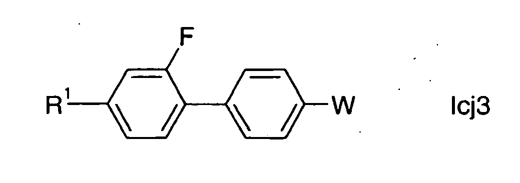 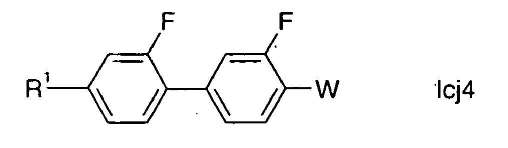 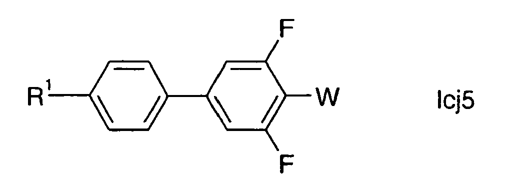 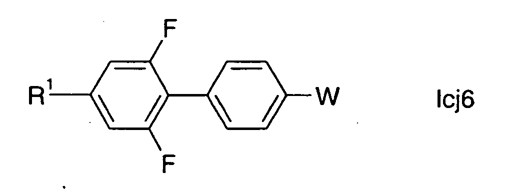 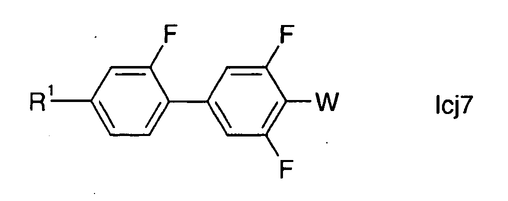 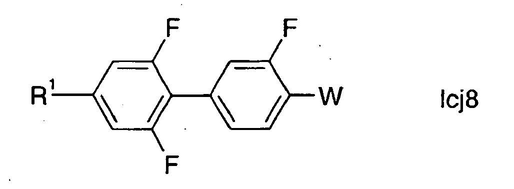 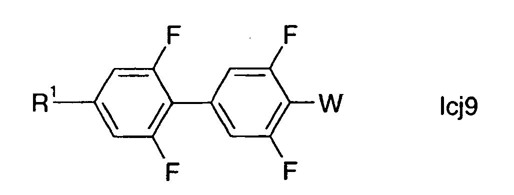 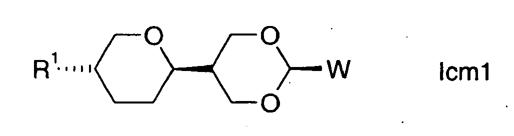 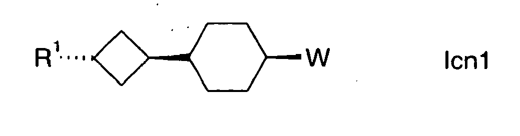 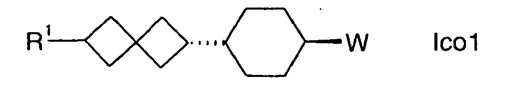 handelt.

7. Verbindungen gemäß Anspruch 5, **dadurch gekennzeichnet, dass** es sich bei den Verbindungen der Teilformel le um mindestens eine Verbindung ausgewählt aus den folgenden Formeln 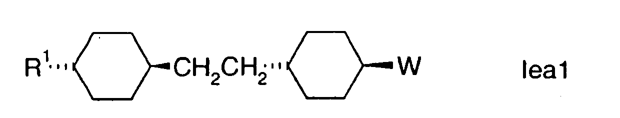 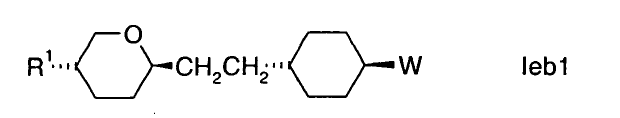 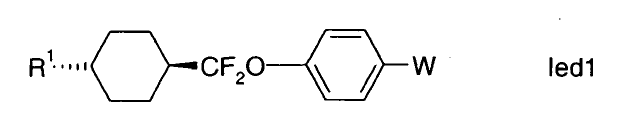 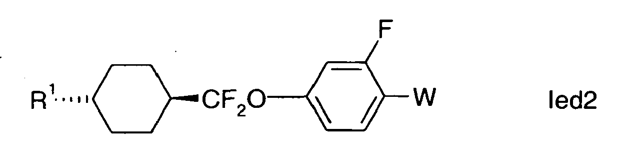 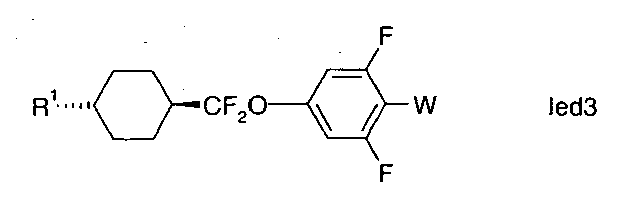 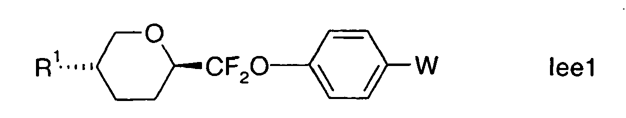 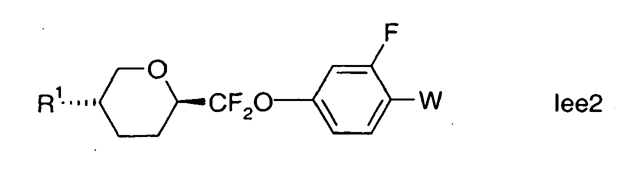 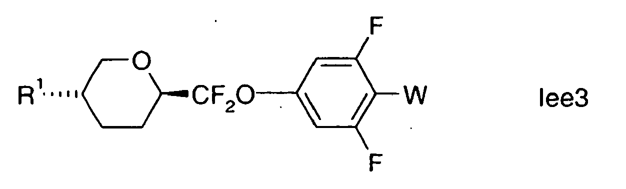 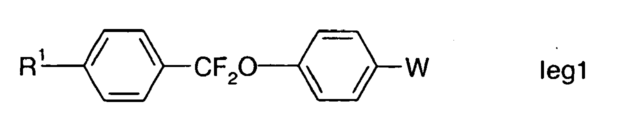 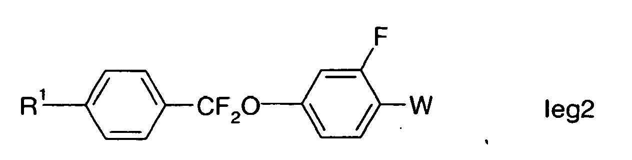 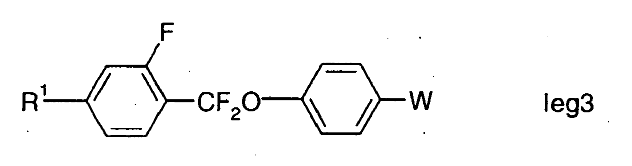 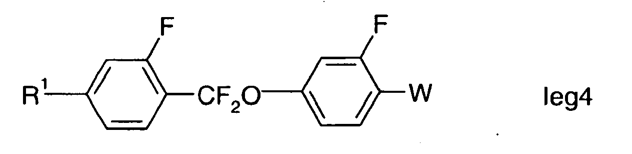 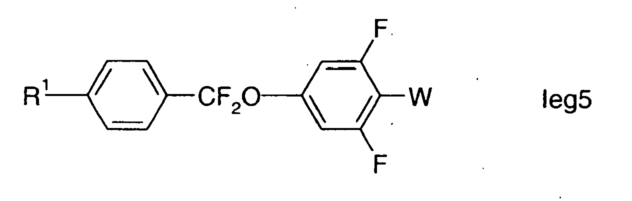 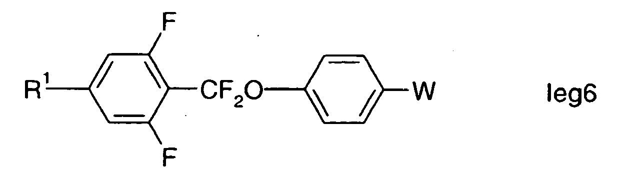 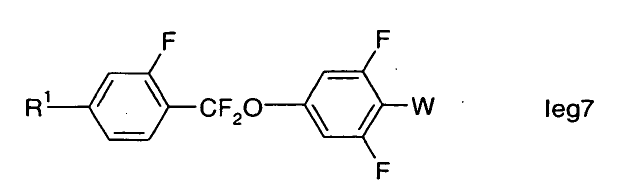 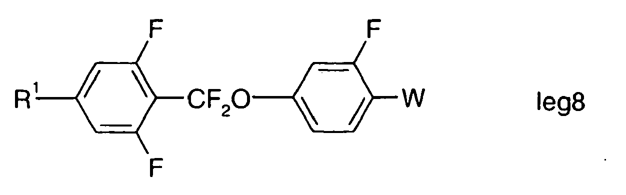 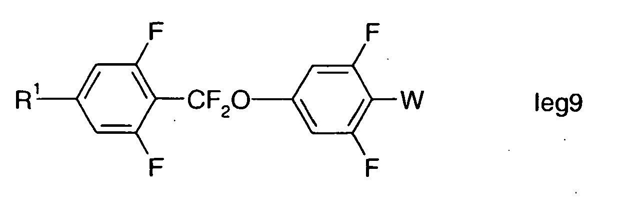 handelt.

8. Verbindungen gemäß einem oder mehreren der vorangehenden Ansprüche, **dadurch gekennzeichnet, dass** der Molekülteil W, gemäß Anspruch 4 definiert, einer der folgenden Teilformeln (1) bis (5) 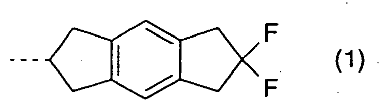 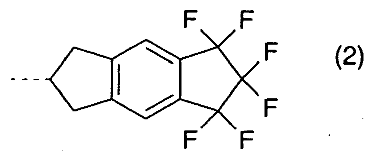 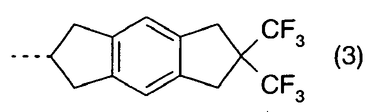 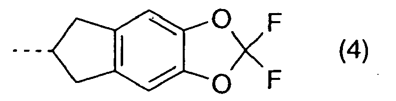 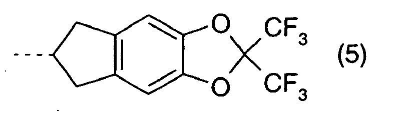 entspricht.

9. Verbindungen gemäß einem oder mehreren der vorangehenden Ansprüche, **dadurch gekennzeichnet, dass** R¹ H, einen linearen Alkyl- bzw. Alkoxyrest mit 1 bis 12 C-Atomen oder einen linearen Alkenyl- bzw. Alkenyloxyrest mit 2 bis 12 C-Atomen bedeutet.

10. Verwendung von Verbindungen der Formel I gemäß einem oder mehreren der vorangehenden Ansprüche als Komponente(n) in flüssigkristallinen Medien.

11. Flüssigkristallines Medium mit mindestens zwei flüssigkristallinen Komponenten, **dadurch gekennzeichnet, dass** es mindestens ein 1,2,3,6,7,8-Hexahydro-s-indacen- oder 6,7-Dihydro-5H-indeno[5,6-d][1,3]dioxol-Derivat gemäß einem oder mehreren der vorangehenden Ansprüche enthält.

12. Flüssigkristall-Anzeigeelement, **dadurch gekennzeichnet, dass** es ein flüssigkristallines Medium gemäß Anspruch 11 enthält.

13. Elektrooptisches Anzeigeelement, **dadurch gekennzeichnet, dass** es als Dielektrikum ein flüssigkristallines Medium gemäß Anspruch 11 enthält.

14. Verfahren zur Herstellung von Verbindungen der Formel I gemäß einem oder mehreren der vorangehenden Ansprüche, **dadurch gekennzeichnet, dass** es einen oder mehrere Verfahrenschritte umfasst, wodurch an ein 5,6-Dibrom- oder ein 5,6-Dihydroxy-Derivat des Indans oder des Benzo[1,3]dioxols in 5,6-Position ein Cyclopentanring angeschlossen wird.

## Claims

1. 1,2,3,6,7,8-Hexahydro-s-indacene and 6,7-dihydro-5H-indeno-[5,6-d]-1,3-dioxole derivatives of the general formula I 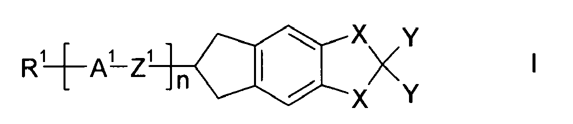 in which
R¹ denotes H, halogen, a linear or branched alkyl radical having 1 to 15 C atoms which is unsubstituted, monosubstituted by CN or CF₃ or at least monosubstituted by halogen and in which, in addition, one or more CH₂ groups may each be replaced, independently of one another, by -O-, -S-, -CO-, -(CO)O-, -CH=CH-, -CH=CF-, -CF=CF- or -C≡C- in such a way that heteroatoms are not linked directly to one another and asymmetrical groups may be present in both orientations,
A¹ in each case, independently of one another, identically or differently, denotes
a) trans-1,4-cyclohexylene, in which, in addition, one or more non-adjacent CH₂ groups may be replaced by -S-, tetrahydropyran-2,5-diyl or 1,3-dioxane-2,5-diyl,
b) 1,4-phenylene, in which one or two CH groups may be replaced by N and in which, in addition, one or more H atoms may be replaced by halogen,
c) a radical from the group 1,4-bicyclo[2.2.2]octylene, spiro[3.3]heptane-2,6-diyl, cyclobutane-1,3-diyl, piperidine1,4-diyl, naphthalene-2,6-diyl, decahydronaphthalene-2,6-diyl and 1,2,3,4-tetrahydronaphthalene-2,6-diyl, in which, in addition, CH may be replaced by N and in which, in addition, one or more H atoms may be replaced by halogen, or
d) 1,4-cyclohexenylene,
X denotes -CH₂-, -CF₂- or -O-,
Y denotes F, Cl, CF₃, CN, NCS, SCN, SF₅ or 2- to 6-C perfluoroalkyl,
Z¹ in each case, independently of one another, in the case of asymmetrical bridging units Z¹ in either of the two orientations, denotes a single bond, -CH₂O-, -(CO)O-, -CF₂O-, -CF=CF-, -CH₂CH₂CF₂O-, -CF₂CF₂-, -CH₂CF₂-, -CH₂CH₂-, -CH=CH-, -CH=CF- or -C≡C-, and
n denotes 0, 1, 2 or 3.

2. Compounds according to Claim 1, **characterised in that**
Y denotes F or CF₃.

3. Compounds according to Claim 1 or 2, **characterised in that**
n is equal to 0.

4. Compounds according to Claim 1 or 2, **characterised in that** n is equal to 1, and the compounds of the formula I are compounds of the following formulae 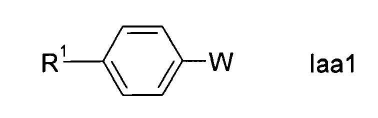 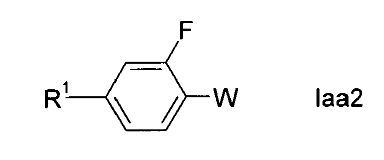 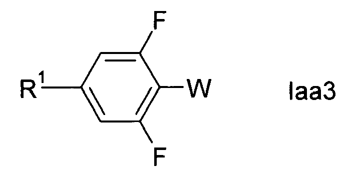 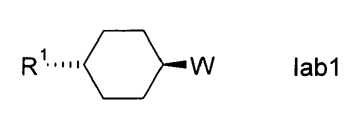 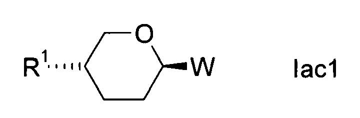 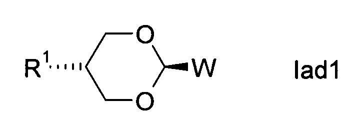 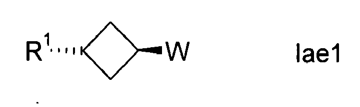 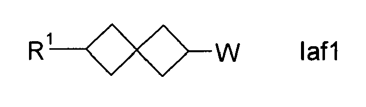 where W stands for the moiety of the formula 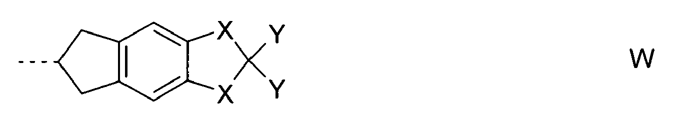 in which X and Y are as defined in Claim 1.

5. Compounds according to Claim 1 or 2, **characterised in that** the compounds of the formula I are compounds having two rings in the mesogenic group R¹-[A¹-Z¹]ₙ-, selected from the group of the compounds of the formulae Ic to If
R¹-A¹-A¹-W Ic
R¹-A¹-A¹-Z¹-W Id
R¹-A¹-Z¹-A¹-W Ie
R¹-A¹-Z¹-A¹-Z¹-W If
where W is as defined in Claim 4.

6. Compounds according to Claim 5, **characterised in that** the compounds of the sub-formula Ic are at least one compound selected from the following formulae

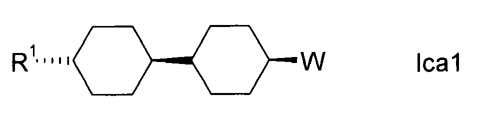

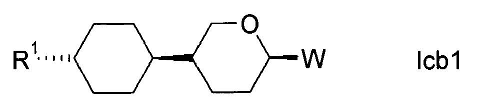

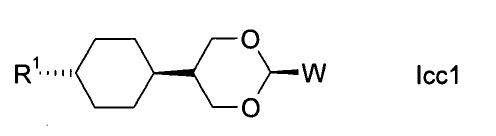

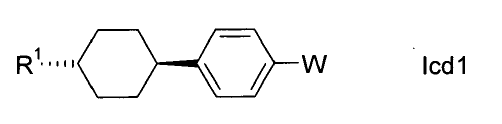

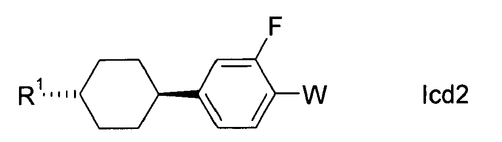

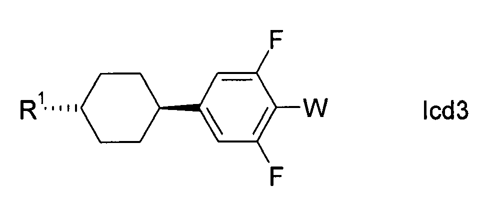

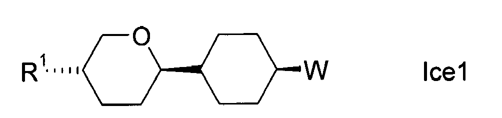

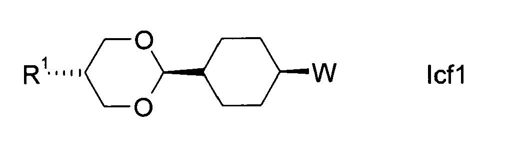

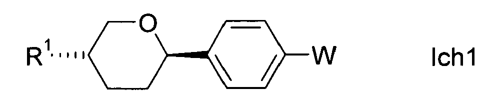

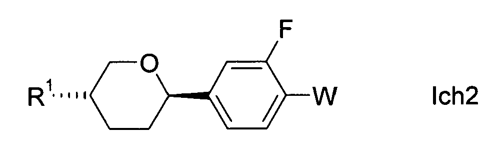

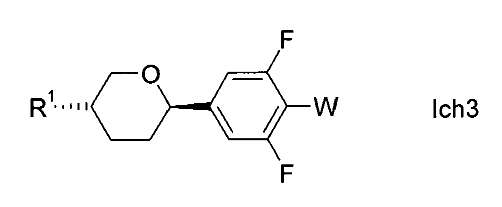

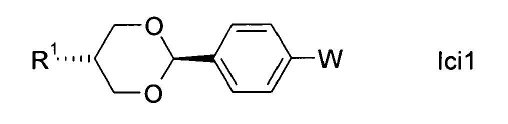

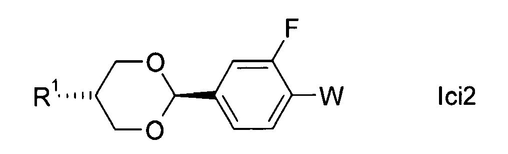

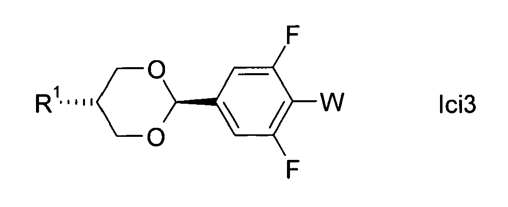

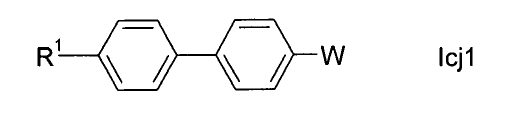

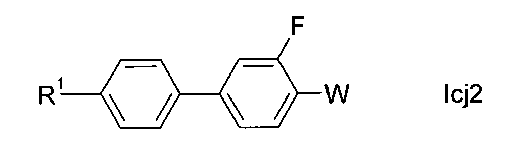

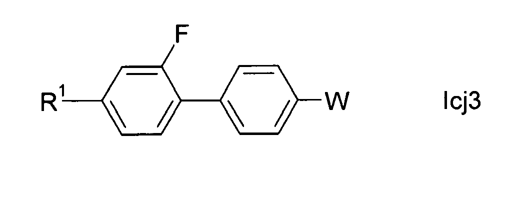

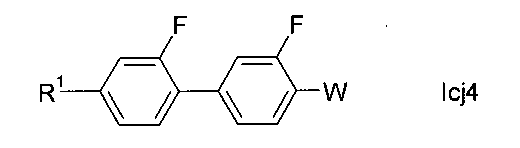

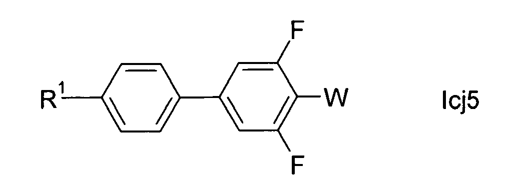

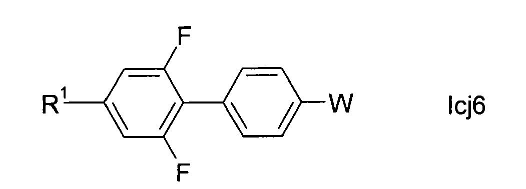

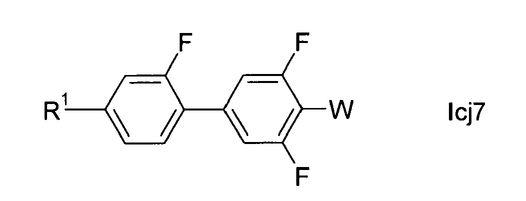

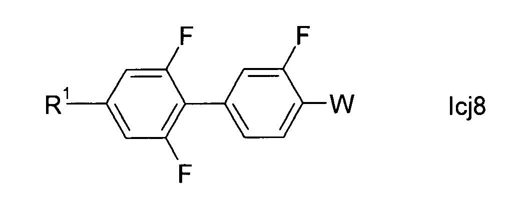

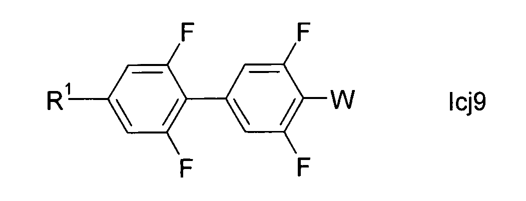

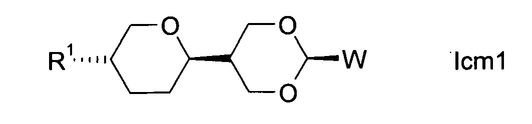

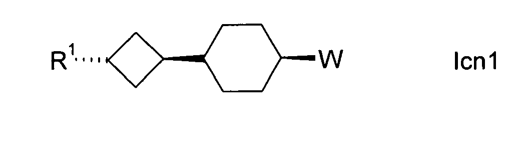

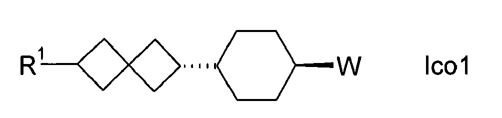

7. Compounds according to Claim 5, **characterised in that** the compounds of the sub-formula le are at least one compound selected from the following formulae

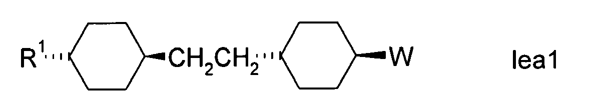

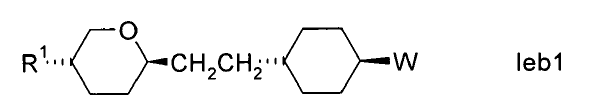

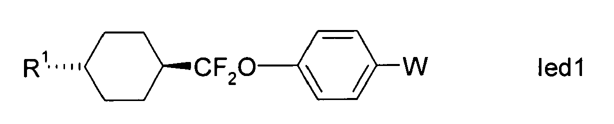

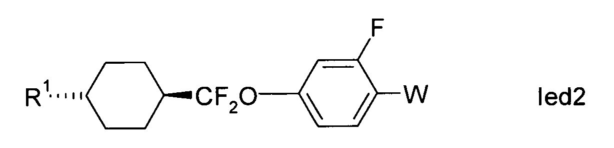

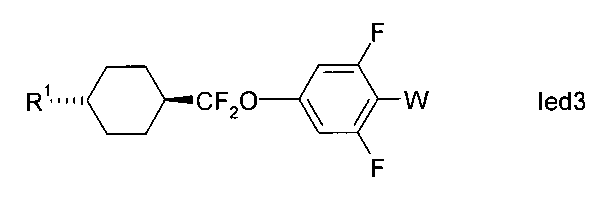

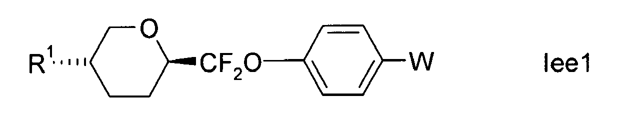

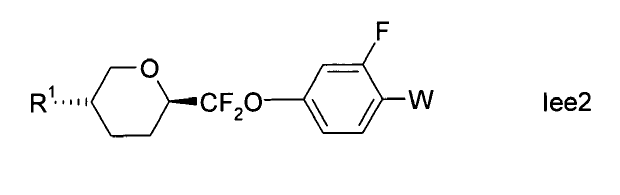

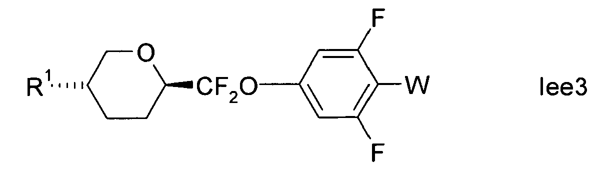

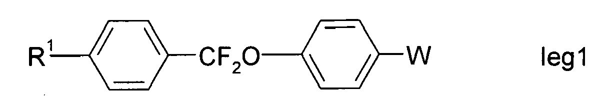

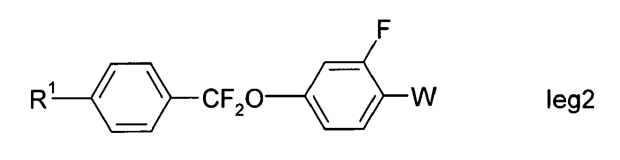

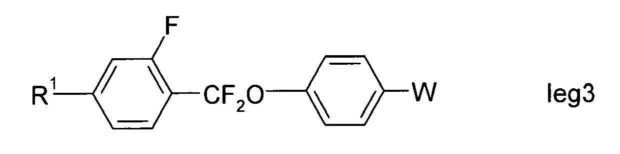

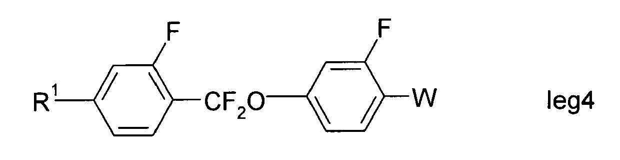

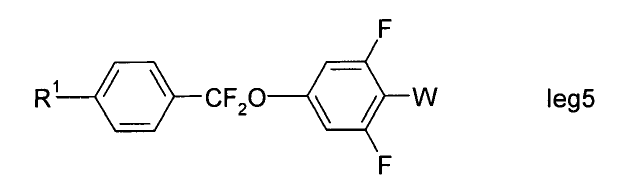

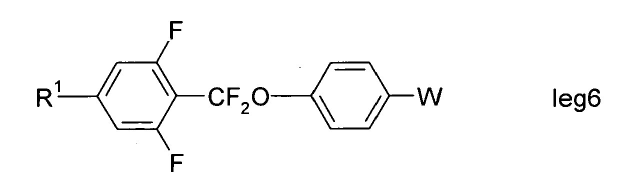

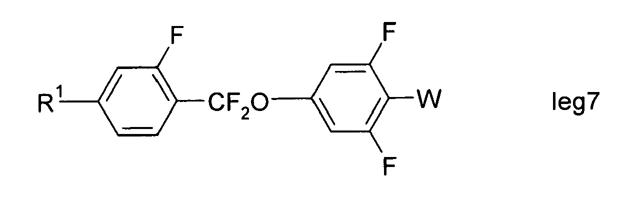

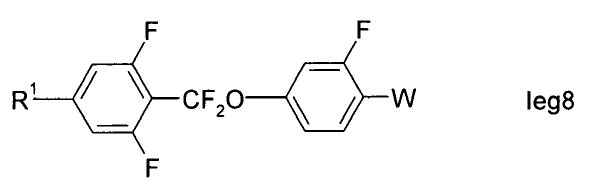

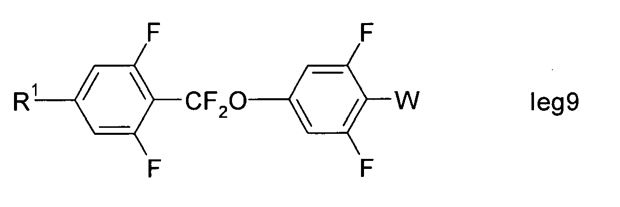

8. Compounds according to one or more of the preceding claims, **characterised in that** the moiety W, defined in accordance with Claim 4, conforms to one of the following sub-formulae (1) to (5)

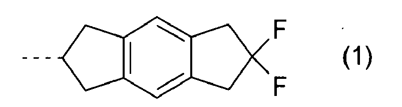

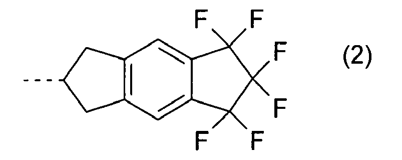

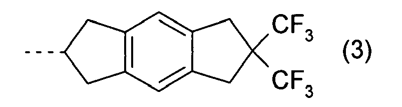

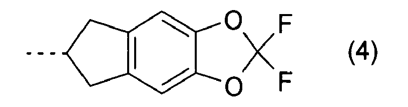

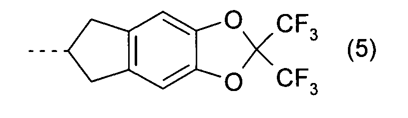

9. Compounds according to one or more of the preceding claims, **characterised in that** R¹ denotes H, a linear alkyl or alkoxy radical having 1 to 12 C atoms or a linear alkenyl or alkenyloxy radical having 2 to 12 C atoms.

10. Use of compounds of the formula I according to one or more of the preceding claims as component(s) in liquid-crystalline media.

11. Liquid-crystalline medium having at least two liquid-crystalline components, **characterised in that** it comprises at least one 1,2,3,6,7,8-hexahydro-s-indacene or 6,7-dihydro-5H-indeno[5,6-d]-1,3-dioxole derivative according to one or more of the preceding claims.

12. Liquid-crystal display element, **characterised in that** it contains a liquid-crystalline medium according to Claim 11.

13. Electro-optical display element, **characterised in that** it contains, as dielectric, a liquid-crystalline medium according to Claim 11.

14. Process for the preparation of compounds of the formula I according to one or more of the preceding claims, **characterised in that** it comprises one or more process steps by means of which a cyclopentane ring is connected in the 5,6-position to a 5,6-dibromo or 5,6-dihydroxy derivative of indane or of benzo-1,3-dioxole.

## Revendications

1. Dérivés 1,2,3,6,7,8-hexahydro-s-indacène et 6,7-dihydro-5H-indéno[5,6-d]-1,3-dioxole de la formule générale I 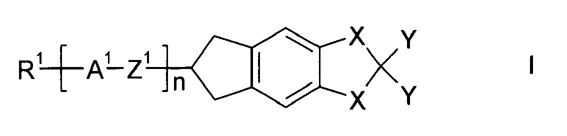 dans laquelle
R¹ représente H, halogène, un radical alkyle linéaire ou ramifié ayant 1 à 15 atomes de C qui est non substitué, monosubstitué par CN ou CF₃ ou au moins monosubstitué par halogène et dans lequel, en addition, un ou plusieurs groupes CH₂ peuvent chacun être remplacés, indépendamment les uns des autres, par -O-, -S-, -CO-, -(CO)O-, -CH=CH-, -CH=CF-, -CF=CF- ou -C≡C- de telle sorte que des hétéroatomes ne soient pas liés directement les uns aux autres et que des groupes asymétriques puissent être présents dans les deux orientations,
A¹ dans chaque cas, indépendamment les uns des autres, de façon identique ou différente, représente
a) trans-1,4-cyclohexylène, dans lequel, en addition, un ou plusieurs groupes CH₂ non adjacents peuvent être remplacés par-S-, tétrahydropyranne-2,5-diyle ou 1,3-dioxane-2,5-diyle,
b) 1,4-phénylène, dans lequel un ou deux groupes CH peuvent être remplacés par N et dans lequel, en addition, un ou plusieurs atomes de H peuvent être remplacés par halogène,
c) une radical parmi le groupe 1,4-bicyclo[2.2.2]octylène, spiro[3.3]heptane-2,6-diyle, cyclobutane-1,3-diyle, pipéridine-1,4-diyle, naphtalène-2,6-diyle, décahydronaphtalène-2,6-diyle et 1,2,3,4-tétrahydronaphtalène-2,6-diyle, dans lequel, en addition, CH peut être remplacé par N et dans lequel, en addition, un ou plusieurs atomes de H peuvent être remplacés par halogène, ou
d) 1,4-cyclohexénylène,
X représente -CH₂-, -CF₂- ou -O-,
Y représente F, Cl, CF₃, CN, NCS, SCN, SF₅ ou 2- à 6-C perfluoroalkyle,
Z¹ dans chaque cas, indépendamment les uns des autres, dans le cas d'unités de pontage asymétriques Z¹ dans l'une ou l'autre des deux orientations, représente une liaison simple, -CH₂O-, -(CO)O-, -CF₂O-, -CF=CF-, -CH₂CH₂CF₂O-, -CF₂CF₂-, -CH₂CF₂-, -CH₂CH₂-, -CH=CH-, -CH=CF- ou -C≡C-, et
n représente 0, 1, 2 ou 3.

2. Composés selon la revendication 1, **caractérisés en ce que** Y représente F ou CF₃.

3. Composés selon la revendication 1 ou 2, **caractérisés en ce que** n est égal à 0.

4. Composés selon la revendication 1 ou 2, **caractérisés en ce que** n est égal à 1, et les composés de la formule I sont des composés des formules suivantes 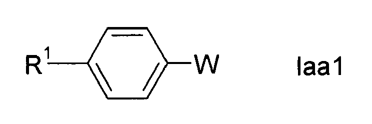 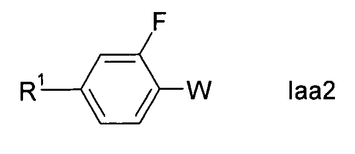 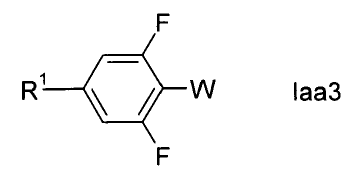 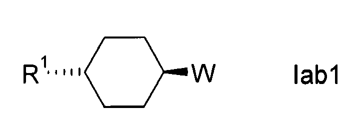 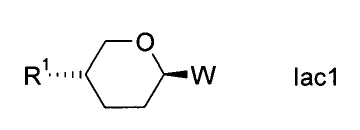 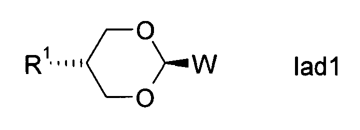 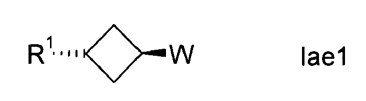 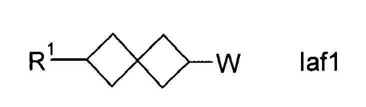 dans lesquelles W représente la moitié de la formule 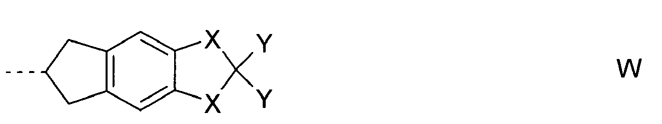 dans laquelle X et Y sont comme défini dans la revendication 1.

5. Composés selon la revendication 1 ou 2, **caractérisés en ce que** les composés de la formule I sont des composés ayant deux cycles dans le groupe mésogène R¹-[A¹-Z¹]ₙ-, choisi parmi le groupe des composés des formules Ic à If
R¹-A¹-A¹-W Ic
R¹-A¹-A¹-Z¹-W Id
R¹-A¹-Z¹-A¹-W Ie
R¹-A¹-Z¹-A¹-Z¹-W If
dans lesquelles W est comme défini dans la revendication 4.

6. Composés selon la revendication 5, **caractérisés en ce que** les composés de la sous-formule Ic sont au moins un composé choisi parmi les formules suivantes

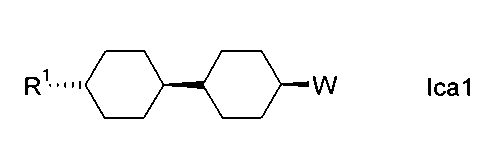

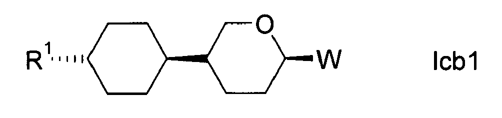

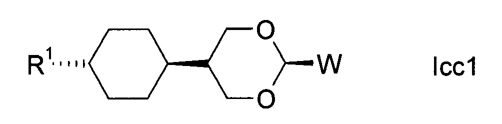

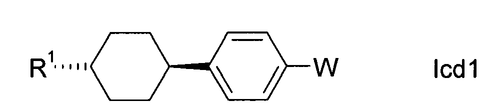

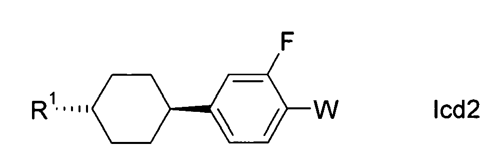

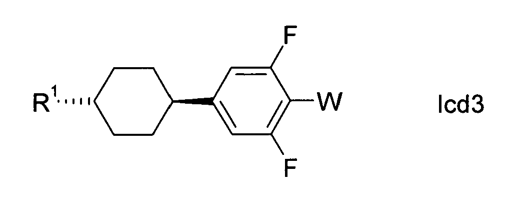

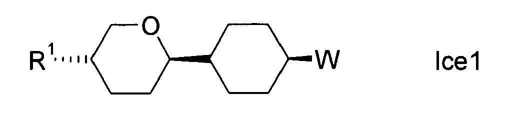

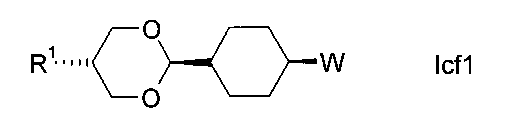

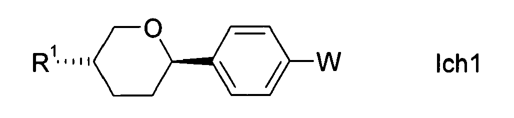

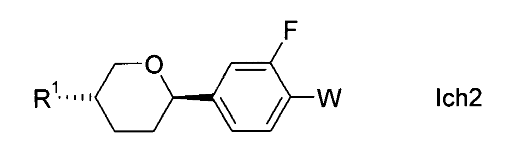

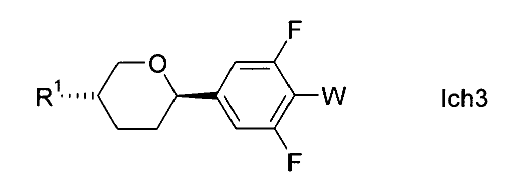

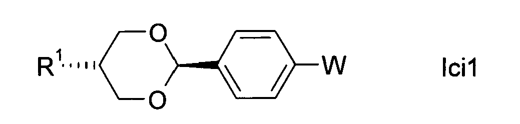

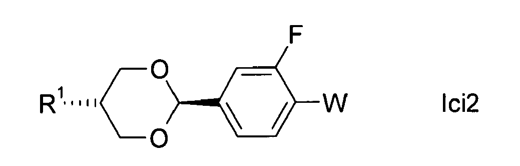

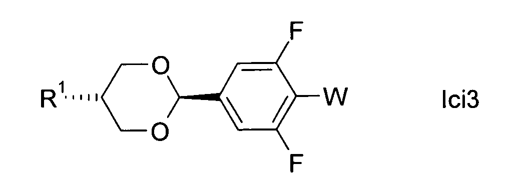

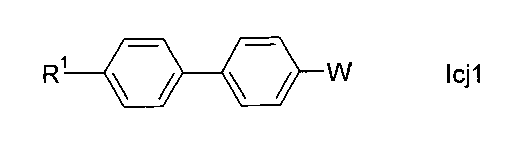

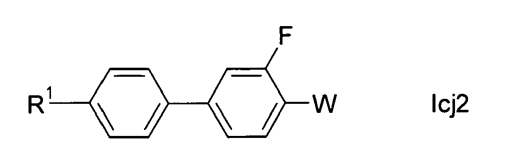

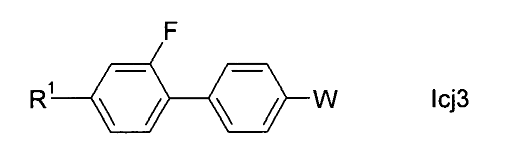

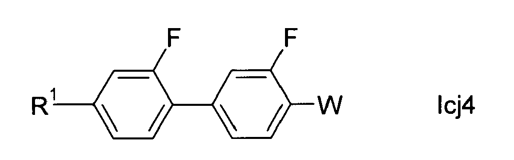

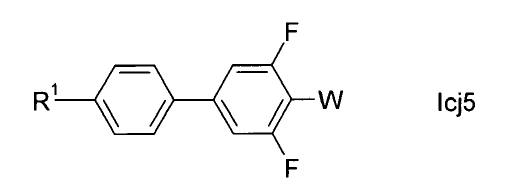

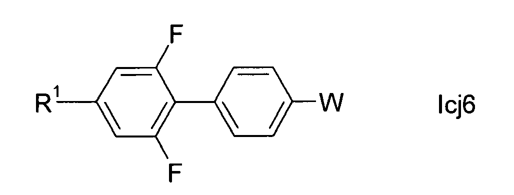

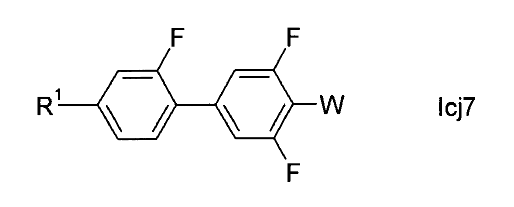

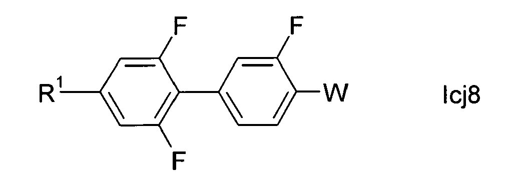

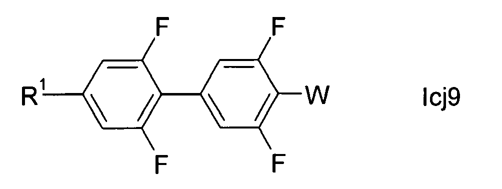

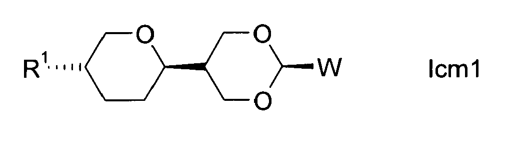

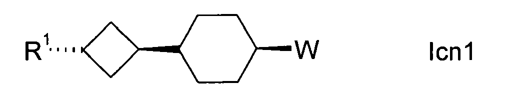

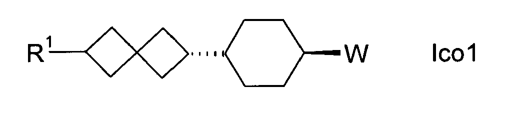

7. Composés selon la revendication 5, **caractérisés en ce que** les composés de la sous-formule le sont au moins un composé choisi parmi les formules suivantes

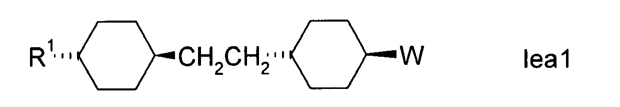

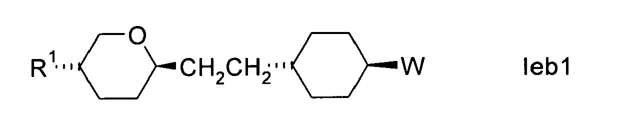

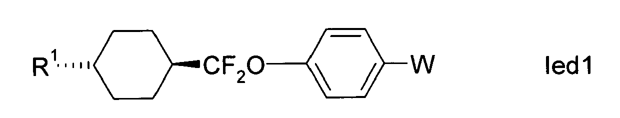

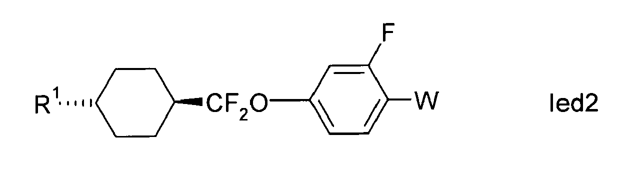

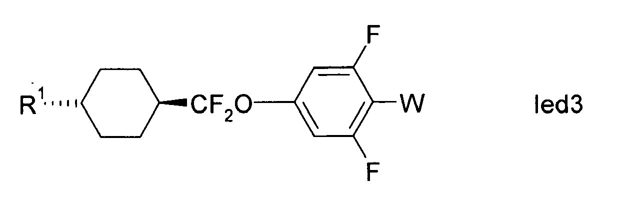

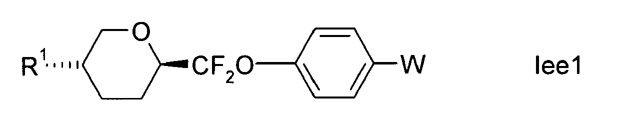

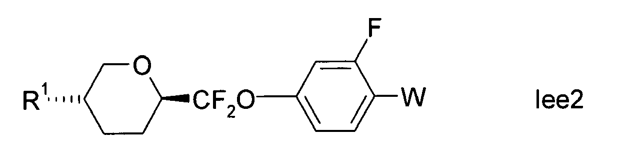

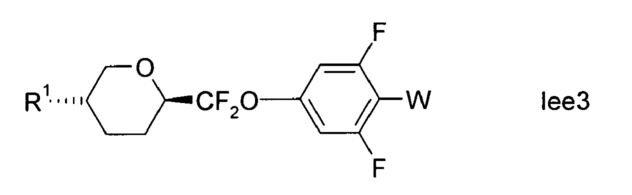

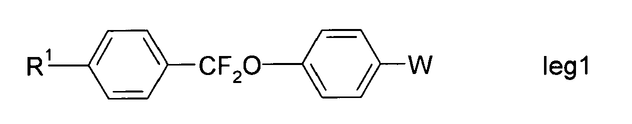

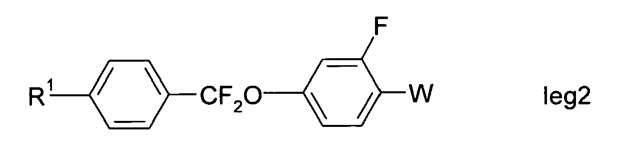

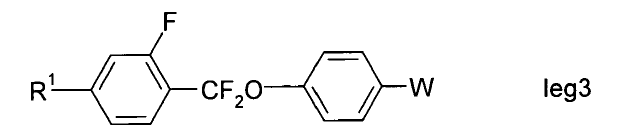

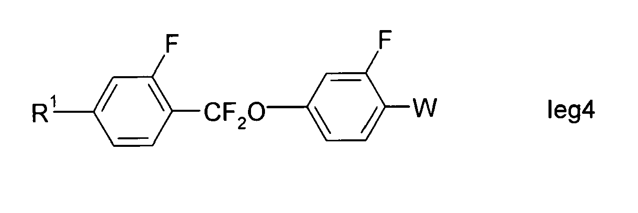

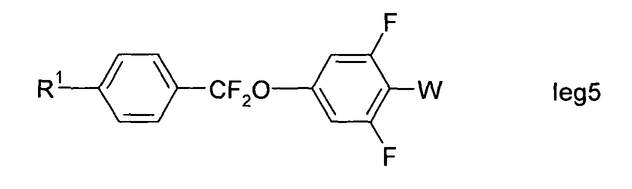

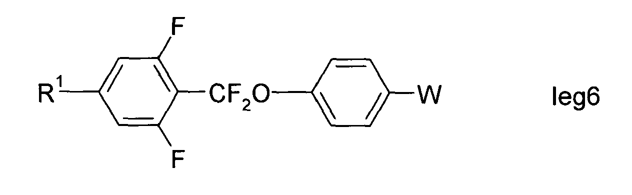

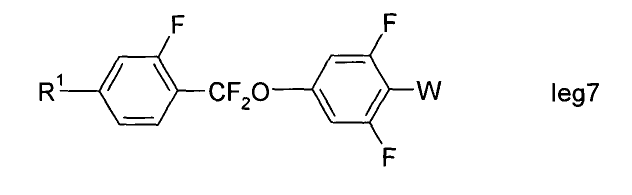

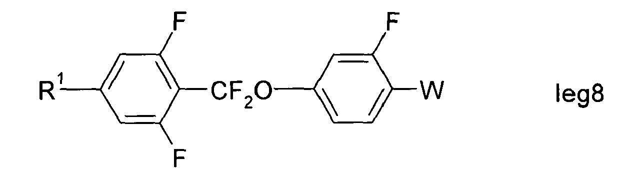

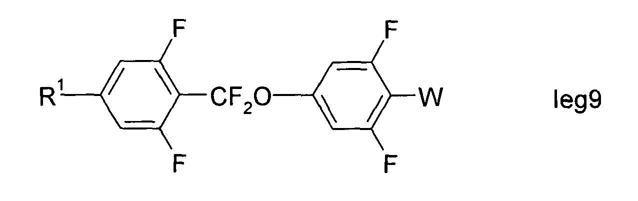

8. Composés selon une ou plusieurs des revendications précédentes, **caractérisés en ce que** la moitié W, définie conformément à la revendication 4, est conforme à une des sous-formules suivantes (1) à (5)

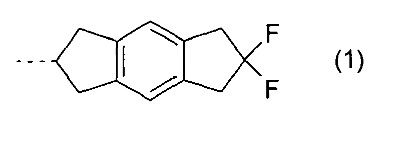

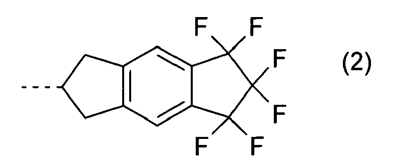

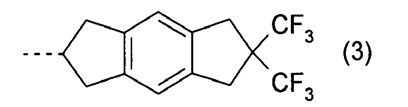

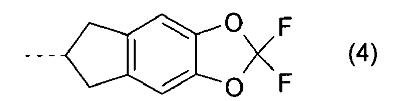

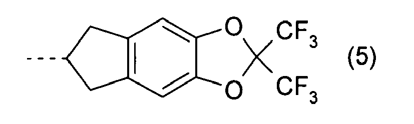

9. Composés selon une ou plusieurs des revendications précédentes, **caractérisés en ce que** R¹ représente H, un radical alkyle ou alcoxy linéaire ayant 1 à 12 atomes de C ou un radical alkényle ou alkényloxy linéaire ayant 2 à 12 atomes de C.

10. Utilisation de composés de la formule I selon une ou plusieurs des revendications précédentes en tant que composant(s) dans des milieux cristallins liquides.

11. Milieu cristallin liquide ayant au moins deux composants cristallins liquides, **caractérisé en ce qu'**il comprend au moins un dérivé 1,2,3,6,7,8-hexahydro-s-indacène ou 6,7-dihydro-5H-indéno[5,6-d]-1,3-dioxole conformément à une ou plusieurs des revendications précédentes.

12. Elément d'affichage à cristal liquide, **caractérisé en ce qu'**il contient un milieu cristallin liquide selon la revendication 11.

13. Elément d'affichage électro-optique, **caractérisé en ce qu'**il contient, en tant que diélectrique, un milieu cristallin liquide selon la revendication 11.

14. Procédé pour la préparation de composés de la formule I conformément à une ou plusieurs des revendications précédentes, **caractérisé en ce qu'**il comprend une ou plusieurs étapes de procédé au moyen desquelles un cycle cyclopentane est connecté dans la position 5,6 à un dérivé 5,6-dibromo ou 5,6-dihydroxy d'indane ou de benzo-1,3-dioxole.
